(19) Europäisches Patentamt
European Patent Office
Office européen des brevets

(11) **EP 3 538 664 B1**

(12) **EUROPÄISCHE PATENTSCHRIFT**

(45) Veröffentlichungstag und Bekanntmachung des Hinweises auf die Patenterteilung:
**07.07.2021 Patentblatt 2021/27**

(21) Anmeldenummer: **17808353.1**

(22) Anmeldetag: **10.11.2017**

(51) Int Cl.:
***C12Q 1/00*** *(2006.01)*

(86) Internationale Anmeldenummer:
**PCT/EP2017/078897**

(87) Internationale Veröffentlichungsnummer:
**WO 2018/087295 (17.05.2018 Gazette 2018/20)**

(54) **KOMPETITIVER TEST EINES ENZYMSUBSTRATS MIT INTERNER KOMPENSATION DER ENZYMAKTIVITÄT**

COMPETITION TEST OF AN ENZYME SUBSTRATE WITH INTERNAL COMPENSATION FOR ENZYME ACTIVITY

TEST DE COMPÉTITION D'UN SUBSTRAT ENZYMATIQUE AVEC COMPENSATION INTERNE DE L'ACTIVITÉ ENZYMATIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.11.2016 DE 102016121553**

(43) Veröffentlichungstag der Anmeldung:
**18.09.2019 Patentblatt 2019/38**

(73) Patentinhaber: **Technische Universität Darmstadt**
**64289 Darmstadt (DE)**

(72) Erfinder:
• SCHMITZ, Katja
64846 Groß-Zimmern (DE)
• WEBER, Christian
22393 Hamburg (DE)
• HÖNES, Joachim
64673 Zwingenberg (DE)
• RAPP, Bastian E.
76135 Karlsruhe (DE)
• WACK, Julia Susanna
64319 Pfungstadt (DE)

(74) Vertreter: **Fuchs Patentanwälte Partnerschaft mbB**
**Westhafenplatz 1**
**60327 Frankfurt am Main (DE)**

(56) Entgegenhaltungen:
**EP-A2- 0 357 400      WO-A1-2013/053953**

• **FREEMAN M K ET AL: "FIBER-OPTIC BIOSENSOR WITH FLUORESCENCE DETECTION BASED ON IMMOBILIZED ALKALINE PHOSPHATASE", BIOSENSORS AND BIOELECTRONICS, Bd. 7, Nr. 1, 1992, Seiten 49-55, XP026706287, ISSN: 0956-5663**
• **KENSLER T W ET AL: "A facile enzymatic technique for the estimation of nanomolar concentrations of N-phosphonacetyl-l-aspartic acid in plasma", JOURNAL OF BIOCHEMICAL AND BIOPHYSICAL METHODS, Bd. 2, Nr. 1-2, 1. Januar 1980 (1980-01-01), Seiten 29-35, XP023512481, AMSTERDAM, NL ISSN: 0165-022X, DOI: 10.1016/0165-022X(80)90071-8 [gefunden am 1980-01-01]**

**Beschreibung**

Die vorliegende Erfindung betrifft ein Verfahren zur Bestimmung der Konzentration eines Analyten in einer Probe, sowie die Verwendung eines Teststreifens und eines Testsystems, das den Teststreifen und einen Detektor umfasst, zur Durchführung des Verfahrens. Außerdem betrifft die Erfindung die Verwendung des Verfahrens zur Bestimmung der Konzentration eines Analyten in einer Probe.

Fibrinogen ist als finaler Faktor in der Gerinnungskaskade von zentraler Bedeutung. Fibrinogen wird von Thrombin gespalten. Dies ist der Schritt in der Blutgerinnungskaskade, der zu Fibrin-Monomeren und durch deren Aggregation zur Bildung des ersten, mechanisch noch labilen Gerinnsels führt.

Bedingt durch Blutverlust (z.B. durch Operationen oder schwere Verletzungen), Blutverdünnung (z.B. durch Infusionstherapie) oder den Verbrauch von Gerinnungsfaktoren (z.B. perioperativ während herzchirurgischer oder komplexer allgemeinchirurgischer Operationen) kann sich ein allgemeines Gerinnungsfaktorendefizit oder ein isoliertes Fibrinogendefizit entwickeln. Dadurch können schwerwiegende Blutungen ausgelöst werden. Die Fibrinogenkonzentration im Blut sinkt in vielen Patienten mit schweren Traumata rasch ab, und geringe Fibrinogenkonzentrationen sind mit erhöhten Sterberaten assoziiert. Es gibt keine Fibrinogenreserven außerhalb des Blutplasmas. Daher kann der Fibrinogenspiegel im Falle eines raschen Abfalls nur unzureichend durch körpereigene Mechanismen normalisiert werden. Die Defizite müssen daher durch externe Zufuhr ausgeglichen werden. Zur Korrektur eines Fibrinogendefizits wurde früher oftmals gefrorenes Frischplasma (Fresh Frozen Plasma, FFP) eingesetzt. Damit ist jedoch stets die Gefahr der Übertragung von Infektionen oder der Induktion eines Nieren- oder Multiorganversagens verbunden. Außerdem muss bei Gabe von FFP ein hohes Volumen (> 15-20 ml/kg Körpergewicht) transfundiert werden, wodurch das ohnehin bereits stark beanspruchte Herz-Kreislauf-System der Patienten noch weiter belastet wird, was dramatische Folgen haben kann. Man geht daher mehr und mehr dazu über, dem Patienten nicht mehr FFP zu transfundieren, sondern nur die wirklich benötigten Substanzen in Form von Gerinnungsfaktorkonzentraten zu ergänzen.

Um Fibrinogen gezielt einsetzen zu können, müsste man während der Operation in Minutenschnelle eine genaue Bestimmung des Fibrinogengehalts durchführen. Aus dem Stand der Technik sind verschiedene Tests zur Bestimmung der Fibrinogenkonzentration bekannt, unter anderem der Clauss-Test, die Thromboelastometrie (z.B. mittels ROTEM@) und die Bestimmung durch Gravimetrie.

Der Standard-Fibrinogentest ist der Test nach Clauss. Durch einen Überschuss an Thrombin wird das Fibrinogen schnell gespalten. Es bildet sich Fibrin, das zu einem Gerinnsel koaguliert, wodurch sich eine Trübung der Probe einstellt, die nachgewiesen werden kann. Die Zeit bis zu einem Schwellenwert der Trübung hängt mit der Fibrinogenkonzentration zusammen, so dass diese Zeit zur Bestimmung der Fibrinogenkonzentration eingesetzt werden kann. Allerdings ist auch die Blutmatrix nicht ohne Einfluss; sowohl Trübungsstärke als auch die Zeit bis zum Schwellenwert werden beeinflusst. Eine höhere Spezifität wäre daher wünschenswert. Der Test benötigt gut geschultes Laborpersonal und eine entsprechende Ausstattung. Im Routinebetrieb ist mit ca. 55 Minuten Bearbeitungszeit zu rechnen. Damit kommen die Ergebnisse in aller Regel zu spät, bzw. bilden nicht mehr die aktuelle Situation ab. In Abhängigkeit von der Blutungsdynamik können 40 Minuten nach Blutentnahme bereits völlig andere Fibrinogenkonzentrationen vorliegen.

Der ROTEM@-Test der Firma TEM International kann im Operationssaal oder auf der Intensivstation durchgeführt werden. Er basiert auf der Thromboelastometrie. Ergebnisse erhält man in ca. 10 Minuten. Allerdings ist der Test bei der Eingreifschwelle von ca. 1 g/L an der unteren Nachweisgrenze und daher ungenau. Er wird zudem gerade durch Maßnahmen bei Operationen wie Infusion von Plasmaexpandern stark beeinflusst. Außerdem ist zu beachten, dass Geräte zur Durchführung der Thromboelastometrie sehr teuer sind und solche Geräte daher meist nur in Uni-Kliniken und anderen äußerst großen Krankenhäusern zu finden sind.

Gravimetrie erfordert eine komplexe Durchführung und noch weit mehr Zeit als der Clauss-Test.

Einen Schnelltest mit hoher Genauigkeit auch im unteren Konzentrationsbereich (< 1,5 g/L) gibt es derzeit nicht. Gerade dieser Konzentrationsbereich ist jedoch bei reduzierten Fibrinogenspiegeln von besonderer Bedeutung. Die aktuelle Leitlinie zum Gerinnungsmanagement bei blutenden Patienten nach Trauma (Rossaint et al., Critical Care (2016) 20:100; Empfehlung 28 auf den Seiten 24 und 25) empfiehlt daher mittels 3 bis 4 g Fibrinogen-Konzentrat eine "blinde" Initial-Therapie durchzuführen und die weitere Fibrinogen-Therapie dann später auf der Grundlage der im Nachhinein erhaltenen Ergebnisse der Bestimmung der Fibrinogenkonzentration anzupassen. Ähnliche Empfehlungen gibt es unter anderem aus den Bereichen Gynäkologie (peripartale Blutungen), Herzchirurgie (Blutung während OP an Herz-Lungen-Maschine), Transplantationschirurgie (insbesondere Leber-Transplantation), aber auch aus der "Inneren Medizin" (Magenulcus-Blutungen). Mangels alternativer Testmethoden zur Bestimmung der Fibrinogenkonzentration wird also in der aktuellen Praxis zwangsläufig in Kauf genommen, dass es aufgrund der "blinden" Initial-Therapie bei der Fibrinogengabe zu Über- oder Unterdosierungen kommt, was jeweils fatale Auswirkungen haben kann.

Es besteht daher ein dringender Bedarf an einem Schnelltest zur Bestimmung der Fibrinogenkonzentration mit hoher Genauigkeit, insbesondere im unteren Konzentrationsbereich (<1,5 g/L).

Freeman und Bachas: "Fiber-optic biosensor with fluorescence detection based on immobilized alkaline phosphatase", Biosensors & Bioelectronics, Bd. 7, 1992, S. 49-55 offenbaren ein Verfahren, bei dem Alkalische Phosphatase als Beispielenzym und 4-Methylumbelliferonphosphat als signalgebendes Substrat eingesetzt wird. Das Verfahren wird von der Enzymaktivität beeinflusst, so dass bei jeder Messung Kalibrierkurven mitgemessen werden müssen.

Kensler et al.: "A facile enzymatic technique for the estimation of nanomolar concentrations of N-phosphonoacetyl-L-aspartic acid in plasma, Journal of Biochemical and Biophysical Methods, Bd. 2, 1980, S. 29-35 betrifft ebenfalls ein Verfahren, das unter Ausnutzung einer Enzyminhibition den Anteil eines Analyten in einer Probe bestimmt. Untersucht wird die Inhibition der AT-Case durch PALA in Gegenwart des kompetitiven Co-Substrats Carbamoylphosphat. Es werden Kalibrierproben im Analyselauf mitgeführt.

WO 2013/053953 A1 betrifft ein Verfahren zur Bestimmung der Konzentration von Beta-Lactam-Antibiotika. Es wird ein Verfahren beschrieben, bei dem der Umsatz eines Reportersubstrats durch eine Beta-Lactamase verringert wird, je höher der Anteil des zu bestimmenden Beta-Lactam-Antibiotikums ist, da dieses als ein "Inhibitor-Substrat" wirkt.

EP 0 357 400 A2 beschreibt eine Testvorrichtung zum Bestimmen der Konzentration eines Analyten in einer wässrigen Flüssigkeitsprobe.

Die bekannten Fibrinogentests basieren auf einem Testprinzip, das sich dadurch auszeichnet, dass durch einen Thrombinüberschuss schnell Fibrin-Monomer erzeugt wird, dessen Polymerisation dann ein Signal erzeugt. Das gemessene Signal kann in einer Trübung der Probe (Clauss-Test), in einer Erhöhung der Viskosität der Probe (Thromboelastometrie) oder in gravimetrisch bestimmbarer Gerinnselbildung bestehen. Aus den oben genannten Gründen eignet sich jedoch keines der bekannten Testverfahren zur Anwendung in der Praxis, da der Clauss-Test und die Gravimetrie mit einem zu hohen Zeitbedarf verbunden sind und die Thromboelastometrie bei den relevanten Fibrinogenkonzentrationen im Bereich <1,5 g/L eine ungenügende Genauigkeit aufweist und die Messwerte durch andere Parameter wie Faktor XIII oder infundierte kolloidale Infusionslösung beeinträchtigt werden.

Gemäß der vorliegenden Erfindung ist es gelungen, diese Probleme zu überwinden. Die Erfindung basiert auf dem Einsatz eines im Vergleich zu den bekannten Verfahren völlig anderen Testprinzips. Dieses Prinzip wird in der vorliegenden Beschreibung am Beispiel der Bestimmung der Fibrinogenkonzentration illustriert, ist jedoch allgemein auf enzymkinetische Bestimmungen von Substratkonzentrationen anwendbar.

Die erfindungsgemäße Lösung besteht in einer Bestimmung der Fibrinogenkonzentration über ein enzymkinetisches Verfahren. Fibrinogen und ein signalerzeugendes, insbesondere chromogenes oder fluorogenes, Substrat werden von Thrombin gespalten. Auch die Generierung eines elektrochemischen Signals ist möglich. Durch die Umsetzung des signalerzeugenden Substrats durch das Enzym wird ein Signalstoff, insbesondere ein Farbstoff, Fluorophor oder Reduktionsmittel erzeugt, dessen Bildung detektiert wird. Die Detektion erfolgt beispielsweise über die Messung der Absorption oder der Fluoreszenz bei einer bestimmten Wellenlänge oder über den Stromfluss bei einem geeignet gewählten Potential.

Fibrinogen und das signalerzeugende Substrat konkurrieren um die Umsetzung durch das Enzym. Je mehr Fibrinogen in der Analyselösung vorhanden ist, desto weniger chromogenes Substrat wird gespalten, d.h. desto flacher der Anstieg der Absorption. Diese Kompetition ist literaturbekannt (z.B. Mathur, Biochemistry 1993, 32, 7568).

Ein derartiger enzymatischer Kompetitionstest hängt direkt von der Enzymaktivität und über deren Temperaturabhängigkeit von der Testtemperatur ab. Darüber hinaus nimmt die Aktivität des Enzyms bei Lagerung des Tests üblicherweise ab. Je nach Zeit und Lagerbedingungen ist die Restaktivität variabel. Auch der pH-Wert und die Messwellenlänge sind Faktoren, die das Testergebnis stark beeinflussen. Man müsste daher alle diese Bedingungen sehr genau konstant halten, um ein zuverlässiges Ergebnis zu erhalten. Da dies besonders für die Enzymaktivität kaum möglich ist, müsste man stattdessen vor der eigentlichen Bestimmung eine Kalibration mit einer bekannten Fibrinogenkonzentration durchführen. Fibrinogen ist allerdings auch nicht stabiler. Vor allem aber würde die Kalibrationslösung stark von der Blutmatrix abweichen, die die Thrombinaktivität wiederum beeinflusst. Als Resultat wäre die Kalibration stark fehleranfällig. Auch würde der zusätzliche Schritt die Handhabung komplexer und langsamer machen.

Aufgrund dieser Schwierigkeiten beruhen die aus dem Stand der Technik bekannten Verfahren zur Bestimmung der Fibrinogenkonzentration nicht auf einem solchen enzymkinetischen Kompetitionstest. Die Erfinder der vorliegenden Erfindung haben jedoch herausgefunden, dass eine zuverlässige Konzentrationsbestimmung über einen solchen Kompetitionstest auch ohne eine komplexe Kalibrierung möglich ist, wenn zwei verschiedene durch die Aktivität des Enzyms erzeugte Signale detektiert und anschließend miteinander verrechnet werden, um die Abhängigkeit von schwer zu kontrollierenden Einflussfaktoren wie Temperatur, pH-Wert und Enzymaktivität zu eliminieren. Theoretisch genau gilt das für die Menge an aktivem Enzym. Bei Temperatur und pH sind gegebenenfalls kleine Reststörungen durch Änderung der $K_M$-Werte möglich. Allerdings ist zu erwarten, dass diese Reststörungen äußerst gering sind, insbesondere wenn signalgebendes Substrat und Analyt ähnlich aufgebaut sind. Auch zusätzliches Enzym oder Inhibitoren/Aktivatoren aus der Probe werden kompensiert, da sie auf beide Messungen gleich wirken. Der entscheidende Vorteil des erfindungsgemäßen Prinzips ist daher die einfache Kompensation von Störungen aller Art. Grundsätzlich gibt es verschiedene Möglichkeiten, dieses erfindungsgemäße Prinzip zu realisieren.

Wenn nicht nur die enzymatische Umsetzung des signalerzeugenden Substrats, sondern auch die enzymatische Umsetzung des Analyten ein messbares Signal erzeugt, besteht eine Möglichkeit der Realisierung des erfindungsgemäßen Prinzips darin, die beiden erzeugten Signale parallel, z.B. bei mehreren Wellenlängen, zu detektieren und die gemessenen Werte geeignet zu verrechnen. Beide Reaktionen hängen von Enzymaktivität und Temperatur sowie weiteren schwer zu kontrollierenden Einflussfaktoren in gleicher Weise ab, da beide Reaktionen in ein und derselben Analyselösung ablaufen. Die Umsetzung des signalerzeugenden Substrats dient dann als interner Standard. Bezieht man die gemessenen Werte daher geeignet aufeinander, so kann die Abhängigkeit von diesen Einflussfaktoren so weit eliminiert werden, dass Fehler aufgrund schwer kontrollierbarer Einflussfaktoren vermieden werden können.

Nicht in allen Fällen entsteht jedoch bei Umsetzung des Analyten durch das Enzym ein messbares Signal. Da das erfindungsgemäße Prinzip jedoch auf der Detektion und Verrechnung zumindest zweier verschiedener durch die Aktivität des Enzyms erzeugter Signale beruht, müssen in Ausführungsformen der Erfindung, in denen die Umsetzung des Analyten kein detektierbares Signal liefert, die beiden Signale durch Umsetzung des signalerzeugenden Substrats erzeugt werden. Hierfür gibt es zumindest die beiden folgenden Möglichkeiten.

Zum einen können die Signale in ein und derselben Analyselösung erzeugt werden. Das setzt jedoch voraus, dass die beiden Signale unterscheidbar sind. Eine Möglichkeit dies zu realisieren besteht darin, dass zwei verschiedene signalerzeugende Substrate eingesetzt werden, die bei Umsetzung durch das Enzym zu unterscheidbaren Signalen führen. Beispielsweise ist es denkbar, dass bei Umsetzung eines ersten signalerzeugenden Substrats durch das Enzym ein erster Farbstoff entsteht und dass bei Umsetzung eines zweiten signalerzeugenden Substrats durch das Enzym ein zweiter Farbstoff entsteht, wobei sich der erste und der zweite Farbstoff beispielsweise in Bezug auf die Wellenlängen ihrer Absorptionsmaxima unterscheiden. Erneut hängen beide Reaktionen von Enzymaktivität und Temperatur sowie weiteren, schwer zu kontrollierenden Einflussfaktoren in gleicher Weise ab, da beide Reaktionen in ein und derselben Analyselösung ablaufen. Bezieht man die gemessenen Werte daher geeignet aufeinander, so kann auch mit diesen erfindungsgemäßen Ausführungsformen die Abhängigkeit von den genannten Einflussfaktoren so weit eliminiert werden, dass Fehler aufgrund schwer kontrollierbarer Einflussfaktoren vermieden werden können.

Zum anderen kann das erfindungsgemäße Prinzip realisiert werden, indem zwei parallele enzymatische Reaktionen bei unterschiedlichen Konzentrationen des signalerzeugenden Substrats durchgeführt werden. Bevorzugt wird in einer solchen Ausführungsform eine Reaktion bei einer sehr hohen Konzentration des signalerzeugenden Substrats durchgeführt. Dadurch ist die gemessene Aktivität des Enzyms praktisch unabhängig von der Gegenwart des Analyten. Mit Hilfe dieser Reaktion kann daher die maximale Anfangsgeschwindigkeit $v_{max}$ der Substratumsetzung bestimmt werden. Die andere Reaktion wird bevorzugt bei einer deutlich geringeren Konzentration des signalerzeugenden Substrats durchgeführt. In diesem Fall besetzt mehr Analyt die Bindungsstellen des Enzyms und es wird dementsprechend weniger signalerzeugendes Substrat umgesetzt. In beiden Fällen ist die Menge des umgesetzten signalerzeugenden Substrats gleichermaßen von Enzymaktivität und Temperatur abhängig. Dividiert man die bestimmten Anfangsgeschwindigkeiten durcheinander, so werden die Abhängigkeiten von der Enzymaktivität und der Temperatur sowie weiteren Einflussfaktoren eliminiert. Dies gilt auch für unterschiedliche Empfindlichkeit der Nachweisreaktion, z.B. durch Stoffe, die beispielsweise die Fluoreszenz des Produktes beeinflussen/löschen. Auch diese Ausführungsformen ermöglichen daher eine schnelle und zuverlässige Bestimmung der Fibrinogenkonzentration mit hoher Genauigkeit auch im unteren Konzentrationsbereich (<1,5 g/L) ohne die Notwendigkeit einer vorherigen Kalibration.

Die Probleme des Stands der Technik werden durch die Gegenstände der Patentansprüche gelöst. Die Probleme werden insbesondere gelöst durch ein Verfahren zur Bestimmung des Gehalts eines Analyten in einer Probe umfassend die folgenden Schritte:

a. Bereitstellen mindestens einer Analyselösung,

i. wobei die mindestens eine Analyselösung jeweils ein Enzym, ein signalerzeugendes Substrat und einen bekannten Anteil der zu analysierenden Probe umfasst, und
ii. wobei das Enzym sowohl den Analyten als auch das signalerzeugende Substrat umsetzen kann, so dass Analyt und signalerzeugendes Substrat um die Umsetzung durch das Enzym konkurrieren,

b. Detektion zweier durch enzymkatalysierte Umsetzung in der mindestens einen Analyselösung erzeugter Signale S1 und S2, wobei die Signale

i. durch enzymkatalysierte Umsetzung einer höheren Konzentration des signalerzeugenden Substrats (Signal S1) und durch enzymkatalysierte Umsetzung einer niedrigeren Konzentration des signalerzeugenden Substrats (Signal S2) in zwei separaten Analyselösungen, oder

ii. durch enzymkatalysierte Umsetzung des signalerzeugenden Substrats (Signal S1) und durch enzymkatalysierte Umsetzung des Analyten (Signal S2), oder

iii. durch enzymkatalysierte Umsetzung eines ersten signalerzeugenden Substrats (Signal S1) und durch enzymkatalysierte Umsetzung eines zweiten signalerzeugenden Substrats (Signal S2)

erzeugt werden,

c. Berechnung eines Umsatzfaktors aus den Signalen, wobei der Umsatzfaktor als Quotient der Anfangsgeschwindigkeiten $v_0(S2)/v_0(S1)$ oder als dessen Kehrwert $v_0(S1)/v_0(S2)$ berechnet wird, und

d. Bestimmung des Gehalts des Analyten in der Probe mit Hilfe des Umsatzfaktors.

Das erfindungsgemäße Verfahren dient der Bestimmung des Gehalts eines Analyten in einer Probe. Als Analyt kommen grundsätzlich sämtliche Substanzen in Frage, die enzymatisch umsetzbar sind. Bevorzugt ist der Analyt ein Peptid, insbesondere ein Polypeptid. Besonders bevorzugt ist der Analyt ein Protein. Sowohl monomere als auch oligomere Proteine sind erfindungsgemäß als Analyten geeignet. In einer besonders bevorzugten Ausführungsform ist der Analyt Fibrinogen.

Optional kann das erfindungsgemäße Verfahren vor dem Schritt des Bereitstellens der mindestens einen Analyselösung den Schritt des Bereitstellens der zu analysierenden Probe umfassen. Der Schritt des Bereitstellens der zu analysierenden Probe ist jedoch bevorzugt nicht Teil des erfindungsgemäßen Verfahrens. Vielmehr kann mit dem Verfahren eine unabhängig bereitgestellte Probe analysiert werden. Bei dieser Probe handelt es sich um die im Hinblick auf den Gehalt des Analyten zu analysierende Probe. Prinzipiell kommen Proben jeglicher Art in Frage. Bevorzugt ist die Probe eine wässrige Probe. Erfindungsgemäß ist eine wässrige Probe eine Probe mit einem Anteil an Wasser von mindestens 25 Gew.-%, weiter bevorzugt mindestens 40 Gew.-%, noch weiter bevorzugt mindestens 45 Gew.-%, noch weiter bevorzugt mindestens 49,5 Gew.-%. Bevorzugt ist die Probe ausgewählt aus der Gruppe bestehend aus Blut, Urin, Speichel, Milch und Schweiß. In bevorzugten Ausführungsformen ist die Probe eine Blutprobe. Das erfindungsgemäße Verfahren kann jedoch auch zur Analyse von Laborlösungen, Lebensmittelextrakten oder von Wasserproben, insbesondere Trinkwasserproben, auf den Gehalt eines bestimmten enzymatisch umsetzbaren Analyten eingesetzt werden.

Die Probe kann vor dem Bereitstellen der Analyselösung bearbeitet werden. Beispielsweise kann die Probe verdünnt werden, wenn zu erwarten ist, dass die Konzentration des Analyten besonders hoch ist. Dieser Verdünnungsfaktor muss bei der Bestimmung des Gehalts des Analyten in der ursprünglichen Probe berücksichtigt werden. Bevorzugt liegt die Konzentration des Analyten in der gemäß Schritt a des Verfahrens eingesetzten Probe in einem Bereich von $0{,}1*K_i$ bis $100*K_i$, bevorzugt von $0{,}2*K_i$ bis $20*K_i$, noch weiter bevorzugt von $0{,}5*K_i$ bis $10*K_i$, wobei $K_i$ die unten beschriebene (Gleichungen 1 bis 4) Michaeliskonstante für die Umsetzung des Analyten durch das Enzym ist, die mit der Umsetzung des signalgebenden Substrats konkurriert. Für die genannten Konzentrationen werden mit dem erfindungsgemäßen Verfahren besonders robuste Ergebnisse erzielt. Die genaue Konzentration des Analyten in der Probe ist vor Durchführung des erfindungsgemäßen Verfahrens nicht bekannt. Diese soll erst durch das Verfahren ermittelt werden. Dem Fachmann wird jedoch in aller Regel ein Konzentrationsbereich bekannt sein, in dem sich die Konzentration des Analyten mit hoher Wahrscheinlichkeit befindet, so dass eine grobe Abschätzung im Vorhinein möglich ist. Je nach Molekulargewicht des Analyten kann die bevorzugte Gewichtskonzentration unterschiedliche Werte annehmen. Für bevorzugte Analyten, insbesondere für Fibrinogen, liegt die Konzentration des Analyten in der bereitgestellten Probe bevorzugt in einem Bereich von 0,05 bis 20 mg/ml, weiter bevorzugt von 0,1 bis 10 mg/ml, weiter bevorzugt von 0,2 bis 5 mg/ml, noch weiter bevorzugt von 0,5 bis 4 mg/ml.

Des Weiteren kann zum Beispiel auch der pH-Wert einer Probe angepasst werden, wenn die ursprünglich bereitgestellte Probe einen pH-Wert aufweist, der die Enzymaktivität in den Analyselösungen hemmen könnte. Bevorzugt liegt der pH-Wert der gemäß Schritt a des Verfahrens eingesetzten Probe in einem Bereich von 6 bis 9, weiter bevorzugt von 6,5 bis 8,5, weiter bevorzugt von 7 bis 8. Besonders bevorzugt liegt der pH-Wert der gemäß Schritt a des Verfahrens eingesetzten Probe in einem Bereich von $\pm1{,}5$, weiter bevorzugt $\pm1{,}0$, noch weiter bevorzugt $\pm0{,}5$ bezogen auf das pH-Optimum des eingesetzten Enzyms. Das pH-Optimum des eingesetzten Enzyms ist erfindungsgemäß der pH-Wert, bei dem das Enzym die höchste Aktivität aufweist.

Bevorzugt ist die bereitgestellte Probe eine flüssige Probe, insbesondere eine wässrige Probe. Es sind jedoch auch feste Proben denkbar, beispielsweise Stuhlproben, Erd- oder Gesteinsproben oder Proben in Pulverform. Lösliche feste Proben können bevorzugt direkt zur Bereitstellung der Analyselösung verwendet werden. Alternativ kann aus einer festen Probe der Analyt extrahiert und der gewonnene Extrakt als Anteil der Probe zur Bereitstellung der Analyselösung verwendet werden. In solchen Ausführungsformen kann die Konzentration des Analyten im Extrakt zur Bestimmung des Gehalts des Analyten in der festen Probe verwendet werden.

Gemäß Schritt a des erfindungsgemäßen Verfahrens wird mindestens eine Analyselösung bereitgestellt, die ein Enzym, ein signalerzeugendes Substrat und eine bekannte Menge der Probe umfasst. Das Enzym kann sowohl den Analyten als auch das signalerzeugende Substrat umsetzen, so dass Analyt und signalerzeugendes Substrat um die Umsetzung durch das Enzym konkurrieren. Mit anderen Worten konkurriert das signalerzeugende Substrat mit der Umsetzung des Analyten durch das Enzym und der Analyt konkurriert mit der Umsetzung des signalerzeugenden Substrats durch das Enzym. Analyt und signalerzeugendes Substrat verhalten sich also zueinander als Kompetitoren.

Die mindestens eine Analyselösung der Erfindung ist eine wässrige Lösung, die die Umsetzung des Analyten und des signalerzeugenden Substrats durch das Enzym erlaubt. Zu diesem Zweck kann die Analyselösung hinsichtlich ihrer Zusammensetzung und Temperatur an die Anforderungen des jeweiligen Enzyms angepasst werden. Insbesondere können der pH-Wert und/oder die Salzkonzentration entsprechend den Anforderungen des jeweiligen Enzyms eingestellt werden. Erfindungsgemäß ist eine wässrige Lösung eine Lösung mit einem Anteil an Wasser von mindestens 50 Gew.-%, weiter bevorzugt mindestens 75 Gew.-%, weiter bevorzugt mindestens 90 Gew.-%, noch weiter bevorzugt mindestens 95 Gew.-%.

Gemäß Schritt a des erfindungsgemäßen Verfahrens wird mindestens eine Analyselösung bereitgestellt. In bevorzugten Ausführungsformen der Erfindung wird genau eine Analyselösung bereitgestellt. In anderen bevorzugten Ausführungsformen werden genau zwei Analyselösungen bereitgestellt. In besonders bevorzugten Ausführungsformen werden eine erste Analyselösung A1 und eine zweiten Analyselösung A2 bereitgestellt, wobei die erste Analyselösung A1 und die zweite Analyselösung A2 jeweils ein Enzym, ein signalerzeugendes Substrat und eine bekannte Menge der Probe umfassen, und wobei die Konzentration des signalerzeugenden Substrats in der zweiten Analyselösung A2 deutlich geringer, bevorzugt höchstens halb so hoch ist wie die Konzentration des signalerzeugenden Substrats in der ersten Analyselösung A1. Solche Ausführungsformen sind insbesondere dann von Vorteil, wenn die enzymatische Umsetzung des Analyten kein detektierbares Signal liefert und keine verschiedenen signalerzeugenden Substrate vorhanden sind, die bei enzymatischer Umsetzung unterscheidbare Signale liefern könnten. In solchen Ausführungsformen sind die Signale S1 und S2, die durch enzymatische Umsetzung des signalerzeugenden Substrats erzeugt werden, als solche nicht voneinander unterscheidbar, so dass die Detektion in zwei separaten Analyselösungen erfolgen muss. Bevorzugt enthalten die verschiedenen Analyselösungen dieselbe Menge an Enzym. Dadurch kann ein zusätzlicher Normierungsschritt vermieden werden. Außerdem ist auf diese Weise ein etwaiger Einfluss der Enzymkonzentration auf zwischen den Reaktionen als konstant zu betrachtende Parameter ausgeschlossen. Bevorzugt enthalten die verschiedenen Analyselösungen dieselbe Menge der Probe. Dadurch kann ein zusätzlicher Normierungsschritt vermieden werden. Außerdem ist auf diese Weise ein etwaiger Einfluss der Konzentration des Analyten auf zwischen den Reaktionen als konstant zu betrachtende Parameter ausgeschlossen. Der Ausdruck *"dieselbe Menge"* bedeutet erfindungsgemäß, dass die Abweichungen nicht mehr als $\pm$ 1% betragen.

Gemäß Schritt a des erfindungsgemäßen Verfahrens umfasst die mindestens eine Analyselösung ein Enzym. Erfindungsgemäß sind sämtliche Enzyme geeignet, die den Analyten und das signalerzeugende Substrat enzymatisch umsetzen können, wobei zumindest durch Umsetzung des signalerzeugenden Substrats ein detektierbares Signal erzeugt wird. Bevorzugt ist das Enzym ausgewählt aus der Gruppe bestehend aus proteinbasierten Enzymen, Ribozymen und Desoxyribozymen. Besonders bevorzugt ist das Enzym ein Protein. Weiter bevorzugt ist das Enzym ausgewählt aus der Gruppe bestehend aus Oxidoreduktasen, Transferasen, Hydrolasen, Lyasen, Isomerasen und Ligasen. Weiter bevorzugt ist das Enzym ausgewählt aus der Gruppe bestehend aus Hydrolasen und Transferasen. Auf Transferasen lässt sich das erfindungsgemäße Prinzip anwenden, sofern ein geeignetes signalerzeugendes Substrat vorhanden ist, bei dem der Transfer einer chemischen Funktionalität zu einem messbaren Signal, insbesondere zur Veränderung von Absorption oder Fluoreszenz, führt und das mit dem Analyten in Konkurrenz treten kann. Bei Hydrolasen wird das Signal bevorzugt durch hydrolytische Abspaltung eines Signalstoffs aus dem signalerzeugenden Substrat erzeugt. Besonders bevorzugt ist das Enzym eine Hydrolase. Noch weiter bevorzugt ist das Enzym eine Peptidase. Noch weiter bevorzugt ist das Enzym eine Serinprotease. Noch weiter bevorzugt ist das Enzym ausgewählt aus der Gruppe bestehend aus Thrombin und Trypsin. Noch weiter bevorzugt ist das Enzym Thrombin.

Erfindungsgemäß umfasst die mindestens eine Analyselösung mindestens ein signalerzeugendes Substrat. Als signalerzeugende Substrate kommen sämtliche Substrate in Frage, die durch das Enzym umgesetzt werden können und durch deren Umsetzung ein nachweisbares Signal erzeugt wird. Bevorzugt wird durch die enzymatische Umsetzung des signalerzeugenden Substrats ein Signalstoff gebildet. Bevorzugt ist der Signalstoff ein Farbstoff. Unter einem Farbstoff im Sinne dieser Erfindung ist ein Stoff zu verstehen, der optisch über Spektrometer oder Photometer nachgewiesen werden kann. Farbstoffe im Sinne dieser Erfindung sind beispielsweise Stoffe, die Licht bestimmter Wellenlänge absorbieren, so dass die Konzentration der Stoffe über die Absorption der Analyselösung bei dieser Wellenlänge bestimmt werden kann. Farbstoffe im Sinne dieser Erfindung können aber auch Fluorophore sein, also fluoreszente Komponenten, die nicht nur Licht einer bestimmten Wellenlänge absorbieren, sondern auch Licht einer im Vergleich zur Absorptionswellenlänge höheren Wellenlängen emittieren, so dass die Konzentration dieser Farbstoffe über das Ausmaß des emittierten Lichtes nachgewiesen werden kann. Fluoreszente Farbstoffe können enzymatisch insbesondere durch Transferasen, Hydrolasen, Lyasen oder Isomerasen erzeugt werden.

In anderen bevorzugten Ausführungsformen enthält das signalerzeugende Substrat zwei molekulare Einheiten, die als Donor und Akzeptor ein FRET-Paar (Förster-Resonanzenergietransfer) bilden. Bevorzugt sind Donor und Akzeptor hinreichend nah zueinander positioniert, um FRET mit hoher Effizienz zu ermöglichen. Der Donor ist dabei ein Fluorophor. Der Akzeptor ist ein Farbstoff, der im Wellenlängenbereich der Emission des Donors Licht absorbieren kann. Bei Anregung des Donors mit Licht geeigneter Wellenlänge wird die aufgenommene Energie auf den Akzeptor übertragen, so dass die Fluoreszenz des Donors stark verringert ist. Der Akzeptor kann ein Fluoreszenzfarbstoff sein. In diesem Fall kann der Akzeptor bei Anregung des Donors Licht der charakteristischen Akzeptoremissionswellenlängen emittieren. Wenn der Akzeptor kein Fluoreszenzfarbstoff ist, wird keine Fluoreszenz beobachtet. Durch enzymatische Spaltung des Substrats werden Donor und Akzeptor voneinander getrennt, wodurch der stark entfernungsabhängige Energietransfer zwischen Donor und Akzeptor gehemmt wird und es zu einer Steigerung der Emission des Donors kommt.

Der Signalstoff muss jedoch nicht zwangsläufig ein Farbstoff sein. Insbesondere sind auch elektrochemisch nachweisbare Signalstoffe erfindungsgemäß. In Ausführungsformen, in denen der Signalstoff elektrochemisch nachweisbar ist, werden bevorzugt signalerzeugende Substrate eingesetzt, die substratgebundene, besonders bevorzugt peptidgebundene p-Aminophenol- und/oder p-Phenylendiaminreste enthalten. Bei geeignetem Potential kann der substratgebundene Rest nicht, das abgespaltene freie Amin dagegen gut oxidiert werden. Solche signalerzeugenden Substrate eignen sich daher insbesondere zum Einsatz mit Hydrolasen. Weiter bevorzugt ist der Einsatz dieser elektrochemischen signalerzeugenden Substrate mit Peptidasen.

Wie oben beschrieben sind Peptidasen besonders bevorzugte Enzyme im Sinne der vorliegenden Erfindung. Besonders bevorzugte signalerzeugende Substrate gemäß der vorliegenden Erfindung sind daher solche, bei denen das Signal durch Spaltung einer Peptidbindung erzeugt wird. Besonders bevorzugt sind solche signalerzeugenden Substrate, bei denen die Spaltung einer Peptidbindung zur Bildung eines Signalstoffs, insbesondere eines Farbstoffs im Sinne der Erfindung führt. Besonders bevorzugt umfasst das signalerzeugende Substrat eine p-Nitroanilin-Gruppe, die über ihre Aminogruppe mit der Carboxylgruppe einer Aminosäure, besonders bevorzugt mit der Carboxylgruppe einer basischen Aminosäure, ganz besonders bevorzugt mit der Carboxylgruppe von Arginin, über eine Peptidbindung verbunden ist. Wird diese Peptidbindung durch das Enzym gespalten, wird p-Nitroanilin freigesetzt. Die Konzentration von p-Nitroanilin kann bei einer Wellenlänge von 405 nm spektroskopisch nachgewiesen werden. P-Nitroanilin ist ein besonders bevorzugter Farbstoff gemäß der vorliegenden Erfindung. Besonders bevorzugte signalerzeugende Substrate gemäß der vorliegenden Erfindung sind N-Benzoyl-D,L-Arginin-p-Nitroanilid (BAPNA) sowie die Oligopeptidsubstrate Ala-Gly-Arg-p-Nitroanilid und p-Tosyl-Gly-Pro-Arg-p-Nitroanilid. Durch Wahl anderer Peptide lassen sich praktisch alle bekannten Peptidasen und ihre natürlichen Substrate auswählen, so dass das Testprinzip auf viele Anwendungen übertragbar ist.

Besonders bevorzugt sind auch solche signalerzeugenden Substrate, bei denen die Spaltung einer Peptidbindung zur Bildung eines Signalstoffs, insbesondere eines fluoreszierenden Farbstoffs im Sinne der Erfindung führt. Besonders bevorzugt umfasst das signalerzeugende Substrat eine Coumaringruppe, insbesondere eine 7-Amino-4-methylcourmaringruppe, die über ihre Aminogruppe mit der Carboxylgruppe einer Aminosäure, besonders bevorzugt mit der Carboxylgruppe einer basischen Aminosäure, ganz besonders bevorzugt mit der Carboxylgruppe von Arginin, über eine Peptidbindung verbunden ist. Wird diese Peptidbindung durch das Enzym gespalten, wird 7-Amino-4-methylcourmarin freigesetzt. Die Konzentration von 7-Amino-4-methylcourmarin kann über die Absorption bei einer Wellenlänge von etwa 340 nm und über die Emission bei einer Wellenlänge von etwa 460 nm spektroskopisch nachgewiesen werden.

Erfindungsgemäß sind auch Ausführungsformen, in denen das signalerzeugende Substrat selbst ein Signalstoff im Sinne der vorliegenden Erfindung ist, wobei dieser Signalstoff durch die enzymatische Umsetzung abgebaut wird. In solchen Ausführungsformen besteht das detektierte Signal also in einer Abnahme der Absorption oder in einer Abnahme der Fluoreszenz. Erfindungsgemäß sind auch Ausführungsformen gemäß denen sowohl das signalerzeugende Substrat als auch der durch enzymatische Umsetzung des signalerzeugenden Substrats gebildete Stoff ein Signalstoff sind, sofern sich deren Signale voneinander unterscheiden lassen. Häufig wird zum Beispiel ein signalerzeugendes Substrat elektromagnetische Strahlung einer bestimmten Wellenlänge absorbieren, während ein durch enzymatische Umsetzung des signalerzeugenden Substrats gebildeter Signalstoff Strahlung einer anderen, insbesondere einer höheren Wellenlänge absorbiert. Auch solche Paare aus signalerzeugendem Substrat und daraus gebildetem Signalstoff sind erfindungsgemäß.

Erfindungsgemäß umfasst die mindestens eine Analyselösung auch einen bekannten Anteil der Probe. Das erfindungsgemäße Prinzip besteht darin, den Gehalt des Analyten in der Probe durch Detektion mindestens zweier durch enzymkatalysierte Umsetzung erzeugter Signale und deren anschließende Verrechnung zu bestimmen. Um Rückschlüsse aus den in der mindestens einen Analyselösung erzeugten Signalen auf den Gehalt des Analyten in der Probe ziehen zu können, muss bekannt sein, welcher Anteil der Probe Eingang in die Analyselösung gefunden hat. Der in der Analyselösung enthaltene Anteil der Probe muss daher für eine erfolgreiche Durchführung des erfindungsgemäßen Verfahrens bekannt sein.

Gemäß Schritt b des erfindungsgemäßen Verfahrens werden zwei durch enzymkatalysierte Umsetzung in der mindestens einen Analyselösung erzeugte Signale S1 und S2 detektiert. Die Art der Signale hängt vom eingesetzten signalerzeugenden Substrat ab. Bevorzugt ist das Signal ein optisch detektierbares Signal, insbesondere eine Änderung der Absorption in der Analyselösung oder ein Fluoreszenzsignal. Eine Änderung der Absorption kann insbesondere in einer Änderung des Extinktionskoeffizienten und/oder in einer Änderung des Absorptionsmaximums bestehen. Ein Fluoreszenzsignal kann insbesondere in einer Änderung der Quantenausbeute und/oder in einer Änderung der Anregungs- und/oder Emissionswellenlänge bestehen.

Ein nachweisbares Signal im Sinne der vorliegenden Erfindung muss nicht zwangsläufig ein optisch detektierbares Signal sein. Erfindungsgemäß sind auch auf andere Weise nachweisbare Signale, insbesondere elektrochemisch detektierbare Signale.

Die Signale werden (i) durch enzymkatalysierte Umsetzung des signalerzeugenden Substrats (Signal S1) und durch enzymkatalysierte Umsetzung des Analyten (Signal S2), oder (ii) durch enzymkatalysierte Umsetzung einer höheren Konzentration des signalerzeugenden Substrats (Signal S1) und durch enzymkatalysierte Umsetzung einer niedrigeren Konzentration des signalerzeugenden Substrats (Signal S2), oder (iii) durch enzymkatalysierte Umsetzung eines ersten signalerzeugender Substrats (Signal S1) und durch enzymkatalysierte Umsetzung eines zweiten signalerzeugender Substrats (Signal S2) erzeugt. Die beiden Signale S1 und S2 werden gemäß den genannten Ausführungsformen also im Hinblick auf schwer zu kontrollierende Parameter wie Temperatur oder Enzymaktivität unter vergleichbaren oder sogar identischen Bedingungen erzeugt. Durch die Detektion zweier Signale und deren anschließende Verrechnung gelingt gemäß der vorliegenden Erfindung eine Eliminierung des Einflusses solcher schwer zu kontrollierenden Faktoren, wodurch eine schnelle und zuverlässige Bestimmung der Konzentration des Analyten mit hoher Genauigkeit ohne die Notwendigkeit einer vorherigen Kalibration ermöglicht wird.

Gemäß bevorzugten Ausführungsformen wird ein Signal S1 durch enzymkatalysierte Umsetzung des signalerzeugenden Substrats und ein Signal S2 durch enzymkatalysierte Umsetzung des Analyten erzeugt. Sofern sich die Signale S1 und S2 in solchen Ausführungsformen voneinander unterscheiden lassen, was aufgrund der voneinander verschiedenen signalerzeugenden Moleküle (signalerzeugendes Substrats einerseits und Analyt andererseits) häufig der Fall sein dürfte, können die Signale in ein und derselben Analyselösung detektiert werden, so dass das Verfahren mit einer einzigen Analyselösung erfolgreich durchgeführt werden kann.

Gemäß weiteren bevorzugten Ausführungsformen wird ein Signal S1 durch enzymkatalysierte Umsetzung einer höheren Konzentration des signalerzeugenden Substrats und ein Signal S2 durch enzymkatalysierte Umsetzung einer niedrigeren Konzentration des signalerzeugenden Substrats erzeugt. In solchen Ausführungsformen können die beiden Signale S1 und S2 an sich nicht voneinander unterschieden werden, da sie auf den gleichen signalerzeugenden Molekülen beruhen. Daher sind in solchen Ausführungsformen zwei separate Analyselösungen vorgesehen, um eine Unterscheidung der Signale S1 und S2 zu ermöglichen.

In solchen Ausführungsformen wird also eine erste Analyselösung A1 und eine zweite Analyselösung A2 bereitgestellt, wobei das Signal S1 in der ersten Analyselösung A1 und das zweite Signal S2 in der zweiten Analyselösung A2 erzeugt werden. Die Begriffe erste und zweite Analyselösung sollen nicht zum Ausdruck bringen, dass die Analysen nacheinander oder gar in einer bestimmten Reihenfolge vorgenommen werden müssen. Vielmehr werden die beiden Analysen bevorzugt parallel durchgeführt. Die erste Analyselösung A1 und die zweite Analyselösung A2 umfassen jeweils ein Enzym, ein signalerzeugendes Substrat und eine bekannte Menge der Probe. Die Konzentration des signalerzeugenden Substrats in der ersten Analyselösung A1 ist höher als die Konzentration des signalerzeugenden Substrats in der zweiten Analyselösung A2. Bevorzugt ist die Konzentration des signalerzeugenden Substrats in der ersten Analyselösung A1 mindestens doppelt so hoch, weiter bevorzugt mindestens dreimal so hoch, weiter bevorzugt mindestens fünfmal so hoch, weiter bevorzugt mindestens zehnmal so hoch, weiter bevorzugt mindestens zwanzigmal so hoch, noch weiter bevorzugt mindestens fünfzigmal so hoch wie die Konzentration des signalerzeugenden Substrats in der zweiten Analyselösung A2. Ein großer Konzentrationsunterschied des signalerzeugenden Substrats in den Analyselösungen trägt zu einer robusten und fehlerarmen Bestimmung der Konzentration des Analyten bei.

Bevorzugt beträgt die Konzentration des signalerzeugenden Substrats in der ersten Analyselösung A1 mindestens 80%, weiter bevorzugt mindestens 90%, weiter bevorzugt mindestens 95%, weiter bevorzugt mindestens 99%, weiter bevorzugt mindestens 99,9% der zur Sättigung des Enzyms benötigten Konzentration. Je höher die Konzentration des signalerzeugenden Substrats, desto robuster ist das gemessene Signal. Unter der zur Sättigung des Enzyms benötigten Konzentration wird in diesem Zusammenhang die Konzentration des signalerzeugenden Substrats in der ersten Analyselösung A1 verstanden, ab der eine Erhöhung der Konzentration des signalerzeugenden Substrats um 10% mit einer Erhöhung des Signals um nicht mehr als 0,1% einhergeht.

Die Konzentration des signalerzeugenden Substrats in der ersten Analyselösung A1 ist also bevorzugt vergleichsweise hoch. Bevorzugt beträgt die Konzentration des signalerzeugenden Substrats in der ersten Analyselösung A1 mindestens $10*K_M$, weiter bevorzugt mindestens $25*K_M$, weiter bevorzugt mindestens $50*K_M$, weiter bevorzugt mindestens $100*K_M$, noch weiter bevorzugt mindestens $200*K_M$, wobei $K_M$ die unten beschriebene (Gleichungen 1 bis 4) Michaeliskonstante ist.

Die Konzentration des signalerzeugenden Substrats in der zweiten Analyselösung A2 kann in einem relativ großen Bereich variiert werden. Mit sehr geringen Werten erhält man zwar sehr hohe Umsatzfaktoren, muss aber berücksichtigen, dass dabei die Umsatzgeschwindigkeit sehr gering und entsprechend schwierig zu messen wird. Bevorzugt liegt daher die Konzentration des signalerzeugenden Substrats in der zweiten Analyselösung A2 in einem Bereich von $1*K_M$ bis $20*K_M$, weiter bevorzugt von $2*K_M$ bis $10*K_M$, ganz besonders bevorzugt von $4*K_M$ bis $8*K_M$, wobei $K_M$ die unten beschriebene (Gleichungen 1 bis 4) Michaeliskonstante ist.

Gemäß weiteren bevorzugten Ausführungsformen wird ein Signal S1 durch enzymkatalysierte Umsetzung eines ersten signalerzeugender Substrats und ein zweites Signal S2 durch enzymkatalysierte Umsetzung eines zweiten signalerzeugender Substrats erzeugt. Diese Ausführungsformen sind den oben beschriebenen Ausführungsformen ähnlich, in denen das Signal S1 durch enzymkatalysierte Umsetzung des signalerzeugenden Substrats und das Signal S2 durch enzymkatalysierte Umsetzung des Analyten erzeugt wird. Allerdings wird gemäß den hier beschriebenen Ausführungsformen im Gegensatz zu den oben beschriebenen Ausführungsformen das zweite Signal nicht durch Umsetzung des Analyten, sondern durch Umsetzung eines zweiten signalerzeugenden Substrats erzeugt. In diesen Ausführungsformen enthält die Analyselösung also sowohl den Analyten als auch zwei verschiedene signalerzeugenden Substrate. Das Verfahren gemäß solcher Ausführungsformen kann in der Regel in einer einzigen Analyselösung durchgeführt werden, vorausgesetzt, dass sich die Signale S1 und S2 voneinander unterscheiden lassen. Solche Ausführungsformen bieten daher die Möglichkeit, das Verfahren in einer einzigen Analyselösung durchzuführen, auch wenn der Analyt bei enzymatischer Umsetzung kein detektierbares Signal liefert. Voraussetzung für diese Ausführungsformen ist jedoch, dass zwei verschiedene signalerzeugende Substrate vorhanden sind, die bei enzymatischer Umsetzung zu unterscheidbaren Signalen führen.

In besonders bevorzugten Ausführungsformen werden weder das Signal S1 noch das Signal S2 durch enzymkatalysierte Umsetzung des Analyten erzeugt. Es ist in anderen Worten besonders bevorzugt, wenn sowohl das Signal S1 als auch das Signal S2 durch enzymkatalysierte Umsetzung mindestens eines signalerzeugenden Substrats erzeugt werden, wobei der Analyt kein signalerzeugendes Substrat ist.

Gemäß Schritt c des erfindungsgemäßen Verfahrens wird aus den detektierten Signalen ein Umsatzfaktor berechnet. Bevorzugt wird der Umsatzfaktor aus den Signalen berechnet, indem aus den Signalen die Anfangsgeschwindigkeiten vo(S1) und vo(S2) der enzymatischen Umsetzung des signalerzeugenden Substrats bestimmt und diese Anfangsgeschwindigkeiten vo(S1) und vo(S2) miteinander verrechnet werden. Der Umsatzfaktor wird als Quotient vo(S2)/vo(S1) oder als dessen Kehrwert vo(S1)/vo(S2) berechnet.

Die Anfangsgeschwindigkeit vo der enzymatischen Umsetzung des signalerzeugenden Substrats ergibt sich für den einfachsten Fall der kompetitiven Inhibition als:

$$v_0 = \frac{v_{max}[S]}{\left(1 + \frac{[I]}{K_i}\right)K_M + [S]}.$$

(Gleichung 1)

Dabei ist $v_{max}$ die maximale Umsetzungsgeschwindigkeit, die dem Produkt aus der Wechselzahl $k_{cat}$ und der Konzentration des aktiven Enzyms [E]o entspricht. [S] ist die Konzentration des signalerzeugenden Substrats. $K_M$ ist die Michaeliskonstante der Umsetzung des signalerzeugenden Substrats durch das Enzym. $K_i$ ist die Michaeliskonstante der Umsetzung des Analyten durch das Enzym. Da der Analyt als Inhibitor der Umsetzung des signalerzeugenden Substrats wirkt, wird $K_i$ erfindungsgemäß auch als Inhibitor-Konstante bezeichnet. [I] ist die zu bestimmende Konzentration des Analyten. Die Ausdrücke "$K_i$" und "$K_I$" werden in der vorliegenden Beschreibung synonym verwendet.

Die Anfangsgeschwindigkeit vo lässt sich aus dem gemessenen Signal bestimmen. Dies ist dem Fachmann bestens bekannt. Da bekannt ist, wieviel signalerzeugendes Substrat eingesetzt wurde ist die Konzentration [S] ebenfalls bekannt. $K_M$ und $K_i$ sind experimentell bestimmbare Größen, die durch Kalibrationsexperimente mit Konzentrationsreihen von signalerzeugendem Substrat und Analyt zu ermitteln sind. Dennoch kann die Konzentration [I] des Analyten nicht ohne weiteres aus der Anfangsgeschwindigkeit vo bestimmt werden, da die maximale Umsetzungsgeschwindigkeit $v_{max}$ des signalerzeugenden Substrats nicht bekannt ist. Wie oben beschrieben ist in Bezug auf $v_{max}$ im Gegensatz zu anderen Parametern wie $K_M$ und $K_i$ eine Bestimmung im Vorfeld durch geeignete Kalibrationsexperimente nicht hilfreich, da $v_{max}$ sehr stark von schwer zu kontrollierenden Einflussfaktoren wie Temperatur, Enzymaktivität und Eigenschaften der komplexen Probenmatrix abhängt. Daher wird erfindungsgemäß aus den detektierten Signalen ein Umsatzfaktor berechnet. Dieser Umsatzfaktor ist weitestgehend unabhängig von den schwer zu kontrollierenden Einflussfaktoren, da diese Faktoren die Messung des ersten Signals S1 und die Messung des zweiten Signals S2 in gleicher Weise beeinflussen und daher durch die Verrechnung der Signale zu einem Umsatzfaktor eliminiert werden können.

Eine besonders einfache Berechnung des Umsatzfaktors ist dann möglich, wenn in einer Reaktion sehr viel signalerzeugendes Substrat und sehr wenig Analyt vorhanden ist, so dass die Anfangsgeschwindigkeit vo der Umsetzung des signalerzeugenden Substrats der maximalen Umsetzungsgeschwindigkeit $v_{max}$ entspricht (z.B. $v_0(S1) = v_{max}$). Wenn die Konzentrationen an Enzym und Analyt in der ersten Analyselösung A1 gleich den Konzentrationen an Enzym und Analyt in der zweiten Analyselösung A2 sind, ergibt sich der Umsatzfaktor U in einem solchen Fall als:

$$U = \frac{v_0(S2)}{v_0(S1)} = \frac{[S]}{\left(1 + \frac{[I]}{K_i}\right)K_M + [S]}$$

(Gleichung 2)

Selbstverständlich kann man anstelle von U auch den Kehrwert 1/U verwenden.

Im Allgemeinen ergibt sich für den Umrechnungsfaktor die Formel:

$$U = \frac{v_0(S2)}{v_0(S1)} = \frac{[S_2]v_{max}}{K_M + K_m\frac{[I]}{K_i} + [S_2]} * \frac{K_M + K_m\frac{[I]}{K_i} + [S_1]}{[S_1]v_{max}} = \frac{[S_2]}{[S_1]} * \frac{K_M + K_m\frac{[I]}{K_i} + [S_1]}{K_M + K_m\frac{[I]}{K_i} + [S_2]}$$

(Gleichung 3)

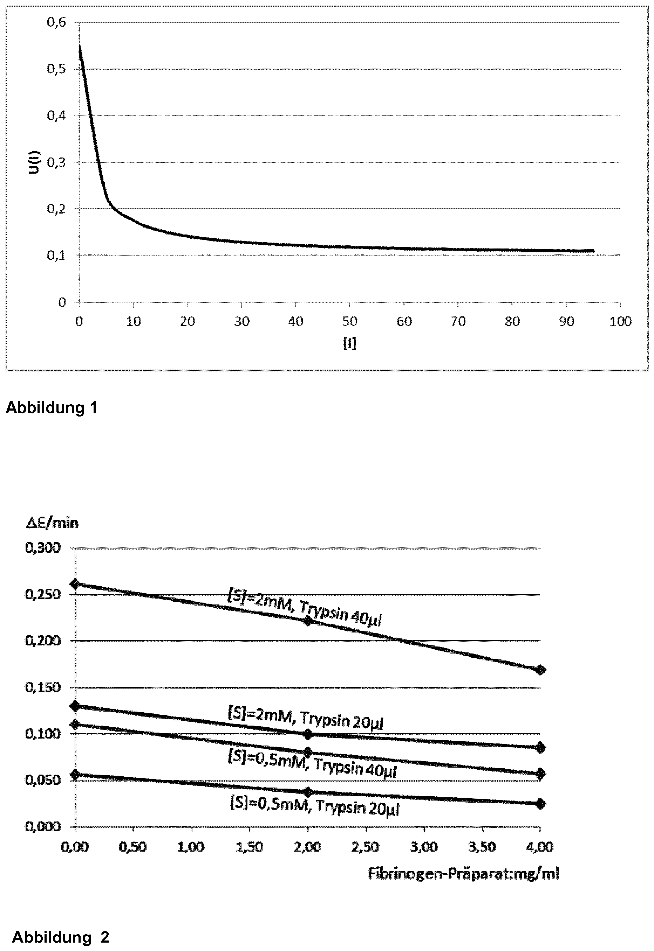

Ohne Analyt ([I] = 0) nimmt U den Wert $U(0) = \frac{[S_2]}{[S_1]} * \frac{K_M + [S_1]}{K_M + [S_2]}$ an. Für große Mengen von Analyt nähert sich U dem Grenzwert $[S_2]/[S_1]$. Die dynamische Breite wird also durch das Konzentrationsverhältnis von erstem und zweitem signalerzeugenden Substrat bestimmt. Je kleiner der Unterschied in den Konzentrationen, desto kleiner die dynamische Breite und desto flacher die Steigung. Der allgemeine Kurvenverlauf von U([I]) ist in Abbildung 1 dargestellt. Der Kehrwert ergibt den inversen Kurvenverlauf mit positiver Steigung.

Für kleine Analyt-konzentrationen [I] verläuft die Funktion U([I]) näherungsweise linear und kann durch die ersten beiden Terme der Taylor-Reihe angenähert werden:

$$\tilde{U}([I]) = \frac{[S_2]}{[S_1]} * \frac{K_M + [S_1]}{K_M + [S_2]} + \frac{[S_2] * K_M}{[S_1] * K_i} * \frac{[S_2] - [S_1]}{(K_M + [S_2])^2}[I]$$

(Gleichung 4)

Die Steigung ist für [I] im Bereich von $K_i$ am steilsten und die Bestimmung der Analytkonzentration in diesem Bereich mit der größten Genauigkeit möglich. Die Bestimmung von U durch Bildung eines Quotienten der beiden Anfangsgeschwindigkeiten vo(S1) und vo(S2) kann also selbst dann zu einer zuverlässigen Bestimmung der Konzentration des Analyten eingesetzt werden, wenn vo(S1) deutlich von $v_{max}$ entfernt ist. Bevorzugt beträgt vo(S1) jedoch mindestens $0{,}5*v_{max}$, weiter bevorzugt mindestens $0{,}8*v_{max}$.

Daher sind die Konzentrationen an Enzym und Analyt in der ersten Analyselösung A1 bevorzugt gleich den Konzentrationen an Enzym und Analyt in der zweiten Analyselösung A2, um eine möglichst einfache Berechnung des Umsatzfaktors zu ermöglichen. In Ausführungsformen, in denen die Konzentrationen an Enzym und Analyt in der ersten Analyselösung A1 nicht gleich den Konzentrationen an Enzym und Analyt in der zweiten Analyselösung A2 sind, müssen weitere Normalisierungsfaktoren in die obige Gleichung eingeführt werden. Dies ist für den Fachmann jedoch rechnerisch unproblematisch. Allerdings ist zu berücksichtigen, dass mit der Verwendung einer unterschiedlichen Analyt- und daher Probemenge auch störende Probebestandteile in unterschiedlicher Konzentration in die Analyselösungen 1 und 2 gelangen. Eine derartige Ausführung ist daher nicht bevorzugt.

Gemäß Schritt d des erfindungsgemäßen Verfahrens wird der Gehalt des Analyten in der Probe mit Hilfe des Umsatzfaktors bestimmt. In den oben angeführten Gleichungen 2 bis 4 zur Bestimmung des Umsatzfaktors U als Quotient aus vo(S2) und vo(S1) wurde im Vergleich zur weiter oben angeführten Gleichung 1 zur Bestimmung der Anfangsgeschwindigkeit vo, der Parameter $v_{max}$ eliminiert. Da der Umsatzfaktor U aus den detektierten Signalen berechnet wird und die Parameter [S], $K_M$ und $K_i$ wie oben beschrieben bekannt sind, kann die Konzentration [I] des Analyten in den Analyselösungen und, da die Analyselösungen einen bekannten Anteil der Probe enthalten, auch der Gehalt des Analyten in der Probe mit Hilfe des berechneten Umsatzfaktors bestimmt werden.

Gemäß besonders bevorzugten Ausführungsformen erfolgt die Bestimmung der Konzentration des Analyten anhand einer empirischen Eichkurve. Eine solche Eichkurve wird bevorzugt erhalten, indem der Umsatzfaktor bei verschiedenen Konzentrationen des Analyten bestimmt wird. Auf diese Weise kann die Abhängigkeit des Umsatzfaktors von der Konzentration des Analyten bestimmt werden, so dass aus dem Umsatzfaktor, der für eine unbekannte Probe erhalten wird, auf die Konzentration des Analyten in dieser Probe geschlossen werden kann.

Besonders robuste Ergebnisse erhält man für $[I] \geq K_i$.

Bevorzugt beträgt die Konzentration $[I]$ des Analyten in den Analyselösungen mindestens $1*K_i$, weiter bevorzugt mindestens $2*K_i$, weiter bevorzugt mindestens $5*K_i$, weiter bevorzugt mindestens $10*K_i$. Die Konzentration $[I]$ des Analyten in den Analyselösungen sollte jedoch auch nicht zu groß sein, da ansonsten die Kompetition mit dem signalerzeugenden Substrat sehr groß wird, wodurch sich eine geringere Signalstärke ergibt. Bevorzugt beträgt die Konzentration $[I]$ des Analyten in den Analyselösungen höchstens $1000*K_i$, weiter bevorzugt höchstens $500*K_i$, noch weiter bevorzugt höchstens $200*K_i$. $K_i$ ist dabei die oben beschriebene Inhibitorkonstante.

Bevorzugt liegt das Verhältnis des Quotienten $[S]/K_M$ zum Quotienten $[I]/K_i$ in der zweiten Analyselösung A2 in einem Bereich von 0,1 bis 10, weiter bevorzugt 0,2 bis 5, weiter bevorzugt 0,5 bis 2, noch weiter bevorzugt 0,8 bis 1,2. Ganz besonders bevorzugt beträgt das Verhältnis des Quotienten $[S]/K_M$ zum Quotienten $[I]/K_i$ in der ersten Analyselösung etwa 1:1. In anderen Worten verhalten sich die Konzentrationen von Analyt und signalerzeugendem Substrat in der zweiten Analyselösung A2 zueinander besonders bevorzugt wie $K_i$ zu $K_M$.

In der ersten Analyselösung A1 hingegen liegt das Verhältnis des Quotienten $[S]/K_M$ zum Quotienten $[I]/K_i$ in einem Bereich von 10 bis 1000, weiter bevorzugt 20 bis 500, weiter bevorzugt 50 bis 200, noch weiter bevorzugt 80 bis 120.

Somit ermöglicht das erfindungsgemäße Verfahren eine schnelle und zuverlässige Bestimmung der Konzentration des Analyten mit hoher Genauigkeit ohne die Notwendigkeit einer vorherigen Kalibration.

Die Durchführung des erfindungsgemäßen Verfahrens kann mit Standard-Laborgerätschaften erfolgen. Bevorzugt werden die Analyselösungen in Küvetten oder in Mikrotiterplatten bereitgestellt. Insbesondere wird auch die Messung in diesen oder vergleichbaren Behältnissen durchgeführt. Dies vereinfacht die Detektion der erzeugten Signale, falls es sich um optisch detektierbare Signale handelt.

Das erfindungsgemäße Verfahren kann jedoch auch mit einem Teststreifen durchgeführt werden. Dies ist insbesondere deshalb vorteilhaft, weil das Verfahren dann problemlos auch durch wenig geübte Personen und sogar direkt am Ort einer Operation, in der Notaufnahme, im OP-Saal, Kreißsaal oder der Intensivstation, zum Beispiel zur Bestimmung der Fibrinogenkonzentration, durchgeführt werden kann.

Ein Teststreifen weist einen mehrschichtigen Aufbau auf. Unter anderem müssen Enzym und Substrate getrennt vorliegen. Der Teststreifen weist mindestens zwei Schichten auf. Bevorzugt weist der Teststreifen mindestens drei, weiter bevorzugt mindestens vier, weiter bevorzugt mindestens fünf, noch weiter bevorzugt mindestens sechs Schichten auf. Allerdings weist der Teststreifen bevorzugt höchstens zehn, weiter bevorzugt höchstens acht Schichten auf. Ansonsten wird der Aufbau sehr komplex und damit fehleranfällig.

Der Teststreifen umfasst mindestens eine Enzymschicht, die das Enzym enthält, und mindestens eine Substratschicht, die das signalerzeugende Substrat enthält. Das Enzym und das signalerzeugende Substrat kommen dabei nicht miteinander in Kontakt, so dass es zu keiner Umsetzung des signalerzeugenden Substrats durch das Enzym kommt. Wird jedoch eine wässrige Probe auf den Teststreifen aufgetragen, diffundiert diese durch die Enzymschicht und die Substratschicht und löst das in den getrennten Schichten des Teststreifens immobilisierte Enzym und signalerzeugende Substrat, die dadurch in Kontakt kommen, so dass eine Analyselösung gemäß der vorliegenden Erfindung gebildet wird. Das in der Analyselösung erzeugte Signal kann dann durch zumindest eine Öffnung beobachtet werden, die optional mit einer transparenten Schutzschicht abgedeckt sein kann.

Je nach Ausführungsform kann der Teststreifen eine oder mehr als eine, bevorzugt zwei, Substratschichten aufweisen. Wenn die enzymatische Umsetzung des Analyten selbst zu einem detektierbaren Signal führt, können wie oben beschrieben das durch enzymkatalysierte Umsetzung des signalerzeugenden Substrats und das durch enzymkatalysierte Umsetzung des Analyten erzeugte Signal zur Berechnung des Umsatzfaktors und damit zur Bestimmung des Gehalts des Analyten in der Probe verwendet werden. Vorausgesetzt, dass diese Signale voneinander unterscheidbar sind, kann die enzymatische Umsetzung des signalerzeugenden Substrats und des Analyten in ein und derselben Analyselösung stattfinden. In solchen Ausführungsformen ist daher eine Substratschicht ausreichend. Dasselbe gilt für Ausführungsformen, in denen zwei verschiedene signalerzeugende Substrate eingesetzt werden, die bei Umsetzung durch das Enzym zu voneinander unterscheidbaren Signalen führen. Auch in solchen Ausführungsformen kann die Umsetzung der beiden Substrate in ein und derselben Analyselösung durchgeführt werden, so dass eine Substratschicht ausreichend ist.

In anderen bevorzugten Ausführungsformen, insbesondere wenn die enzymatische Umsetzung des Analyten selbst nicht zu einem detektierbaren Signal führt und wenn nur ein signalerzeugendes Substrat zur Verfügung steht, werden zwei getrennt voneinander auslesbare Substratschichten benötigt, da die beiden zu detektierenden Signale nicht voneinander unterschieden werden können. In solchen Ausführungsformen weist der Teststreifen daher mindestens zwei voneinander getrennt auslesbare Substratschichten, bevorzugt genau zwei voneinander getrennt auslesbare Substratschichten auf. Bevorzugt enthalten die Substratschichten unterschiedliche Mengen an signalerzeugendem Substrat. Weiter bevorzugt enthält eine Substratschicht eine hohe Menge an signalerzeugendem Substrat und die andere Substratschicht eine geringe Menge an signalerzeugendem Substrat. Besonders bevorzugt ist die Menge des signalerzeugenden Substrats in der einen Substratschicht mindestens doppelt so hoch, weiter bevorzugt mindestens dreimal so hoch, weiter bevorzugt mindestens fünfmal so hoch, weiter bevorzugt mindestens zehnmal so hoch, weiter bevorzugt mindestens zwanzigmal so hoch, noch weiter bevorzugt mindestens fünfzigmal so hoch wie die Menge des signalerzeugenden Substrats in der anderen Substratschicht. Ein großer Mengenunterschied des signalerzeugenden Substrats in den verschiedenen Substratschichten trägt zu einer robusten und fehlerarmen Bestimmung der Konzentration des Analyten bei.

Es ist wichtig, dass die beiden Substratschichten so voneinander getrennt sind, dass es auch bei Diffusion der Probe durch die Schichten nicht zu einer Vermischung kommt. Die verschiedenen Substratschichten sind daher im Teststreifen bevorzugt nebeneinander angeordnet, während die Anordnung von Enzymschicht und Substratschicht dagegen "in Serie", also übereinander bzw. untereinander, erfolgt, so dass eine Vermischung von Substrat und Enzym durch die diffundierende Probe gewährleistet ist.

Bevorzugt enthält der Teststreifen höchstens zwei Enzymschichten, weiter bevorzugt genau eine Enzymschicht. Auch in Ausführungsformen, in denen der Teststreifen zwei Substratschichten aufweist, ist bevorzugt nur eine Enzymschicht vorgesehen. In solchen Ausführungsformen ist es vorteilhaft, wenn die Enzymschicht oberhalb der Substratschichten angeordnet wird, so dass die auf den Teststreifen aufgebrachte Probe zunächst die Enzymschicht passiert und erst anschließend in die nebeneinander unterhalb der Enzymschicht angeordneten Substratschichten diffundiert. Dadurch kann eine Vermischung der erzeugten Analyselösungen verhindert werden.

Der Teststreifen kann zusätzlich zur Enzymschicht und zur Substratschicht weitere Schichten enthalten. Bevorzugt enthält der Teststreifen eine Auftragsschicht, auf die die Probe aufgetragen wird. Optional kann unterhalb der Auftragsschicht eine Abtrennschicht vorgesehen sein, die insbesondere zelluläre Bestandteile zurückhält, bevor die Probe in die Enzym- und Substratschichten diffundiert. Dies ist insbesondere dann von Vorteil, wenn die Probe eine Blutprobe ist. Der mehrschichtige Aufbau des Testreifens kann durch eine oder mehrere Klebeschichten und/oder durch eine die anderen Schichten umgebende Hülle zusammengehalten werden. In bevorzugten Ausführungsformen kann eine solche Hülle auch als Auftragsschicht fungieren. Der Teststreifen kann zusätzlich eine Trägerschicht umfassen, auf der der übrige Schichtverbund aufgebracht ist. Eine solche Trägerschicht erleichtert die Handhabung des Teststreifens, da sie es ermöglicht, den Teststreifen zu handhaben, ohne dass der übrige Schichtaufbau mit seinen zum Teil empfindlichen Schichten berührt werden muss.

Erfindungsgemäß ist auch die Verwendung eines Testsystems umfassend einen Teststreifen sowie einen Detektor zur Detektion der Signale S1 und S2 zur Durchführung des erfindungsgemäßen Verfahrens. Bevorzugt ist der Detektor ein Photometer, besonders bevorzugt ein batteriebetriebenes Photometer. Unter den Begriff Photometer fallen auch Fluoreszenzmessgeräte. Das Testsystem kann des Weiteren eine Recheneinheit zur Berechnung des Umsatzfaktors aus den detektierten Signalen und zur Bestimmung der Konzentration des Analyten in der Probe enthalten. Des Weiteren kann das Testsystem eine Ausgabeeinheit enthalten, mit deren Hilfe der Benutzer die ermittelte Konzentration des Analyten ablesen kann.

In einer anderen erfindungsgemäßen Anwendung, in der eines oder beide Signale elektrochemischer Natur sind, ist der Detektor ein Amperemeter oder Voltmeter, bevorzugt ein Amperemeter. Der Detektor kann weiterhin mit Bauteilen zur Signalverstärkung ausgestattet sein. Das Testsystem kann des Weiteren eine Recheneinheit zur Berechnung des Umsatzfaktors aus den detektierten Signalen und zur Bestimmung der Konzentration des Analyten in der Probe enthalten. Des Weiteren kann das Testsystem eine Ausgabeeinheit enthalten, mit deren Hilfe der Benutzer die ermittelte Konzentration des Analyten ablesen kann.

Erfindungsgemäß ist auch die Verwendung des erfindungsgemäßen Verfahrens zur Bestimmung des Gehalts eines Analyten in einer Probe.

Erfindungsgemäß ist außerdem die Verwendung eines Kits zur Durchführung des erfindungsgemäßen Verfahrens. Das Kit umfasst mindestens eine Enzymzubereitung und mindestens eine Zubereitung mindestens eines signalerzeugenden Substrats. Dabei umfasst die Enzymzubereitung das oben beschriebene Enzym und die Zubereitung des signalerzeugenden Substrates das oben beschriebene signalerzeugenden Substrat.

Die Enzymzubereitung und/oder die mindestens eine Zubereitung mindestens eines signalerzeugenden Substrats kann ein feste Zubereitung, beispielsweise ein Lyophilisat oder ein Pulver, oder eine Zubereitung in flüssiger Form, beispielsweise eine Lösung, sein. Flüssige Zubereitungen können Lösungen in geeigneten Puffern oder deionisiertem und/oder sterilem Wasser umfassen. Diese und weitere geeignete Zubereitungen und deren Herstellung sind dem Fachmann bekannt.

Das Kit stellt vorzugsweise Anweisungen zur Durchführung des erfindungsgemäßen Verfahrens bereit.

Die mindestens eine Zubereitung mindestens eines signalerzeugenden Substrats kann vorzugsweise zwei oder mehrere verschiedene signalerzeugende Substrate enthalten. Diese verschiedenen signalerzeugenden Substrate führen bei enzymatischer Umsetzung zu unterscheidbaren Signalen.

Vorzugsweise umfasst das Kit zwei Zubereitungen mindestens eines signalerzeugenden Substrats, wobei die Zubereitungen das mindestens eine signalerzeugende Substrat jeweils in unterschiedlichen Konzentrationen enthalten können. Die Konzentration des signalerzeugenden Substrats in der ersten Zubereitung kann dabei vorzugsweise mindestens doppelt so hoch sein wie die Konzentration des signalerzeugenden Substrats in der zweiten Zubereitung.

Weiterhin bevorzugt kann das Kit zwei Zubereitungen mindestens eines signalerzeugenden Substrats umfassen, wobei das signalerzeugende Substrat in den beiden Zubereitungen verschieden ist.

Weiterhin kann das Kit vorzugsweise mindestens eine Küvette und/oder mindestens eine Mikrotiterplatte umfassen. Die bei Verwendung des Kits bzw. bei Durchführung des erfindungsgemäßen Verfahrens durchzuführenden Messungen können mit laborüblichen Geräten wie Fluoreszenzspektrometern oder Mikrotiterplattenlesern durchgeführt werde. Dem Fachmann sind entsprechende Messverfahren bekannt.

Bevorzugt stellt das Kit die genannten Zubereitungen jeweils in getrennten Behältnissen bereit. Die Behältnisse sind vorzugsweise verschließbar, insbesondere verschließbare Küvetten und/oder Mikrotiterplatten.

Beispiele

Im Folgenden soll die Erfindung anhand einiger Ausführungsbeispiele illustriert werden.

**Kompetition BAPNA/Fibrinogen um Spaltung durch Trypsin**

In einer ersten Versuchsreihe wurde die Kompetition von BAPNA (N-Benzoyl-D,L-Arginin-p-Nitroanilid) und Fibrinogen um Spaltung durch das Enzym Trypsin untersucht. Spaltung von BAPNA durch Trypsin führt zur Freisetzung von p-Nitroanilin und dadurch zu einer Zunahme der Absorption bei 405 nm.

Einfluss der Enzymaktivität

Zunächst wurde die Enzymaktivität variiert, um eine Abnahme der Enzymaktivität während der Lagerung des Tests oder eine Inhibition des Enzyms durch in der Probe enthaltene Faktoren zu simulieren. Eine Messreihe wurde bei einer Enzymkonzentration von 60 μg Trypsin pro ml der Analyselösung durchgeführt, während die Trypsinkonzentration in der anderen Analyselösung lediglich 40 μg/ml betrug. Außerdem wurden zwei verschiedene BAPNA-Konzentrationen (0,2 mM und 2 mM) getestet. Die Fibrinogenkonzentration wurde zwischen 0 mg/ml und 4 mg/ml mit Abstufungen bei 1 mg/ml, 2 mg/ml und 3 mg/ml variiert. Die Messungen wurden bei 25°C durchgeführt.

Mit Zunahme der Fibrinogenkonzentration nimmt die gemessene Geschwindigkeit der BAPNA-Umsetzung ab. Bei niedriger BAPNA-Konzentration ist dieser Effekt relativ stärker. Bei einer sättigenden Konzentration würde man unabhängig vom Fibrinogen $v_{max}$ messen. Eine Erhöhung der Enzymkonzentration führt zu einer proportionalen Erhöhung der Umsatzgeschwindigkeit bei allen Konzentrationen von signalgebendem Substrat und Analyt. Eine Abnahme der Enzymaktivität während der Lagerung des Tests oder eine Inhibition durch die Probe würde dementsprechend über die erniedrigte Aktivität zu einer stark überhöhten Fibrinogen-Bestimmung führen. Dieses Ergebnis zeigt, dass eine enzymkinetische Bestimmung der Konzentration des Analyten in einer Probe empfindlich durch eine Abnahme der Enzymaktivität während der Lagerung des Tests oder eine Inhibition des Enzyms durch in der Probe enthaltene Faktoren beeinflusst wird, so dass ein solcher aus dem Stand der Technik bekannter Test nicht zur Bestimmung der Fibrinogenkonzentration mit hoher Genauigkeit geeignet ist.

Einfluss der Messwellenlänge

Es wurde eine weitere Messreihe bei einer Trypsinkonzentration von 40 μg/ml unter den oben genannten Bedingungen durchgeführt, mit der Ausnahme, dass bei einer Wellenlänge von 425 nm anstelle der oben genannten 405 nm gemessen wurde.

Durch die Abweichung der Wellenlänge wurden deutlich verringerte Werte gemessen, was im Ergebnis zu einer deutlichen Überschätzung der Fibrinogenkonzentration führen würde.

Einfluss der Temperatur

Es wurde eine weitere Messreihe bei einer Trypsinkonzentration von 40 μg/ml unter den oben genannten Bedingungen durchgeführt, mit der Ausnahme, dass die Temperatur 29°C anstelle der oben genannten 25°C betrug.

Die Erhöhung der Temperatur hatte eine Steigerung der Enzymaktivität um ungefähr 20% zur Folge. Dadurch würde eine solche Temperatursteigerung eine geringere Fibrinogenkonzentration vorspiegeln.

Einfluss von Temperatur und Enzymaktivität auf den Umsatzfaktor

Am Beispiel der Kompetition von BAPNA und Fibrinogen um die Umsetzung durch Trypsin konnte gezeigt werden, dass auch der Einfluss großer Unterschiede in Enzymkonzentration, Messwellenlänge und Temperatur durch die Berechnung eines Umsatzfaktors aus den Signalen zweier Messungen mit unterschiedlicher Konzentration des signalgebenden Substrates eliminiert werden können.

Die Abbildungen 1 und 2 zeigen das Prinzip am Beispiel unterschiedlicher Substrat- und Enzymkonzentrationen. Abbildung 2 zeigt, dass sowohl Substrat- als auch Enzymkonzentration die Fibrinogen-abhängige Umsatzgeschwindigkeit beeinflussen.

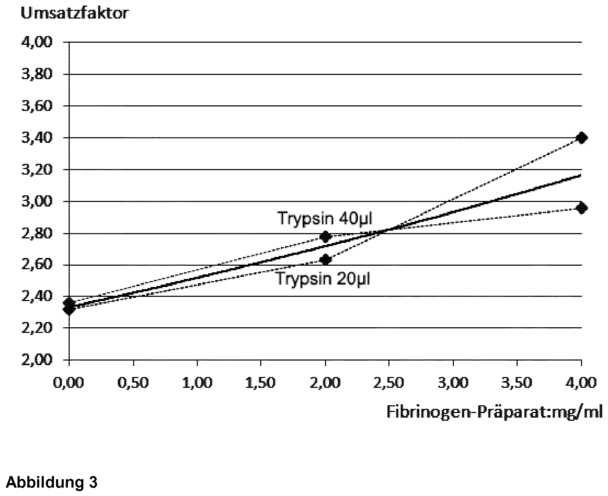

Dividiert man die Umsatzgeschwindigkeit bei hoher Substratkonzentration durch die bei niedriger, so erhält man Umsatzfaktoren, die nur noch von der Fibrinogenkonzentration abhängen (Abbildung 3).

Theoretische Begründung bei kompetitiver Interaktion

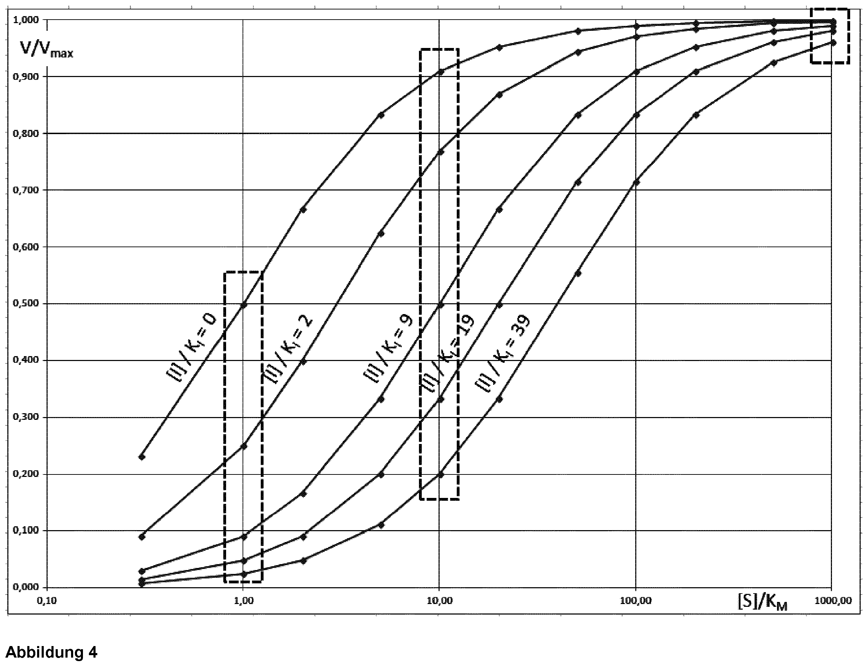

Wenn der Analyt und das signalgebende Substrat mit dem Enzym ein schnelles kompetitives Vorgleichgewicht eingehen und wir als Signal die anfängliche Umsatzgeschwindigkeit des Substrates beobachten, dann können wir die Umsetzung des Analyten vernachlässigen. Dann dürfen wir den Analyten als Inhibitor behandeln und die bekannte Gleichung 1 für kompetitive Hemmung verwenden. Trägt man nun die normierte Umsatzgeschwindigkeit $V/V_{max}$ gegen die normierte Substratkonzentration $[S]/K_M$ (logarithmische Skala) auf, dann ergeben sich abhängig von der normierten Analytkonzentration $[I]/K_I$ verschiedene Kurven, die eine Differenzierung der Analytkonzentration erlauben. Dies ist in Abbildung 4 gezeigt.

Für die hohe Substratkonzentration wählt man bevorzugt eine so hohe Substratkonzentration, dass nahezu unabhängig vom Analyten annähernd $v_{max}$ erreicht wird. Im Beispiel gelingt das bei $[S]/K_M = 1000$ sehr gut (Abbildung 4, Rechteck rechts oben). Mit geringeren Konzentrationen (z.B. $50 \cdot K_M$) erhält man eine entsprechend höhere Variation der Anfangsgeschwindigkeitswerte aber immer noch einen nützlichen Bezugswert zur Errechnung der Umsatzfaktoren.

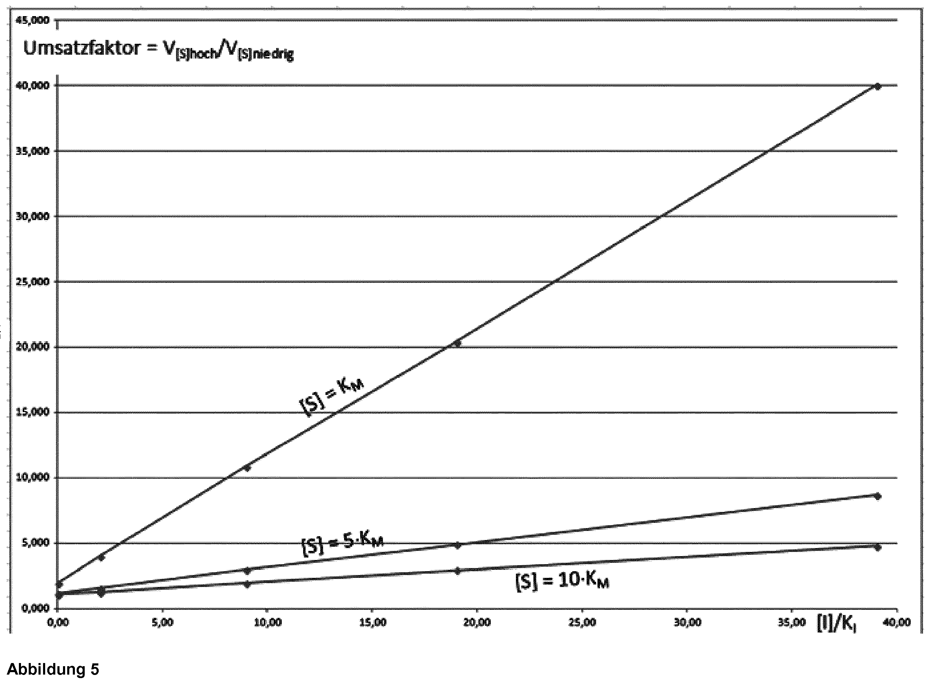

Mit der niedrigeren Substratkonzentration kann man die Differenzierung der Analyt-konzentrationen etwas mehr zu den höheren Werten (Abbildung 4, Rechteck mittig) oder zu den niedrigeren Werten (Abbildung 4, Rechteck links) verschieben. Man erhält so zwar sehr hohe Umsatzfaktoren (Abbildung 5), muss aber berücksichtigen, dass dabei die Umsatzgeschwindigkeit bei der niedrigen Substratkonzentration sehr gering und entsprechend schwierig zu messen wird. Bevorzugt sind daher niedrige Substratkonzentrationen zwischen $1 \cdot K_M$ und $20 \cdot K_M$.

Die Qualität der Differenzierung hängt auch von der normierten Analytkonzentration $[I]/K_I$ ab. Für eine gute Differenzierung ist $[I] \geq K_I$ vorteilhaft. Bei vielen Enzymen ist $K_I$ von den Testbedingungen, z.B. von pH oder Ionenstärke abhängig und man kann entsprechend optimieren. Man kann auch eine passende Probeverdünnung oder-aufkonzentrierung vornehmen oder schließlich ein Enzym mit geeigneter $K_I$ suchen oder gentechnisch generieren.

Es gibt viele weitere Arten der Inhibition wie nicht-kompetitiv, unkompetitiv, gemischt oder auch irreversibel. In manchen Fällen gelten solche Charakterisierungen auch nur innerhalb bestimmter Konzentrationsbereiche von Inhibitor und Substrat. Bei gut bindenden Inhibitoren wird auch die Bedingung des reversiblen Vorgleichgewichtes durch zu langsame Dissoziation des Inhibitors verletzt; kompetitive Bindung ergibt dann nicht-kompetitive Inhibition (J. Hönes, Habilitationsschrift Universität Stuttgart 1985, S.59-61).

Deswegen muss betont werden, dass das erfinderische Prinzip nicht auf den oben geschilderten theoretischen Fall der kompetitiven Inhibition beschränkt ist. Unabhängig von der Art der enzymkinetisch bestimmten Inhibition ist es immer dann anwendbar, wenn Substrat- und Analytumsatz sich gegenseitig beeinflussen. In solchen Fällen kann ein Bezug von zwei Messungen bei niedriger und hoher Substratkonzentration aufeinander die variable Enzymaktivität eliminieren. Man arbeitet dann mit rein empirischen Kalibrationskurven von Umsatzfaktor gegen Analytkonzentration.

**Beispiel Thrombin/Fibrinogen / Fluoreszenzmessung**

In einer 96er-Mikrotiterplatte wurden Puffer, Substrat und Fibrinogen vorgelegt. Das Enzym wurde mit dem zum TECAN-Reader zugehörigen Injektor injiziert. Die Versuchsbedingungen ergeben sich aus den folgenden Angaben.

| | |
|---|---|
| Substrat konz. | Z-Gly-Pro-Arg-Aminomethylcumarin 10mM in DMSO |
| Substrat verd. | Z-Gly-Pro-Arg-Aminomethylcumarin 1mM in DMSO |
| Testpuffer | 50mM Tris, 250mM NaCl, 0,1% PEG6000, pH 8,0 |
| Hämocomplettan | 100mg/ml dest. Wasser $\cong$ 44mg/ml Fibrinogen |
| Enzymlösung | 1U/ml in 10mM HEPES, 0,1% Rinder-Serumalbumin, pH 6,4 |
| Temperatur | 30C |

**Pipettierschema**

| | Well Nr. | | | | | | | | | | | | V in µl |
|---|---|---|---|---|---|---|---|---|---|---|---|---|---|
| | 1 | 2 | 3 | 4 | 5 | 6 | 7 | 8 | 9 | 10 | 11 | 12 | |
| Substrat konz. | 5 | / | 5 | / | 5 | / | 5 | / | 5 | / | 5 | / | |
| Substrat verd. | / | 5 | / | 5 | / | 5 | / | 5 | / | 5 | / | 5 | |
| Testpuffer | 85 | 85 | 75 | 75 | 80 | 80 | 83 | 83 | 84 | 84 | 85 | 85 | |
| Hämocomplettan | 0 | 0 | 10 | 10 | 5 | 5 | 2 | 2 | 1 | 1 | 0 | 0 | |
| Enzymlösung | Injektion jeweils 10 µl zum Start der Reaktion | | | | | | | | | | | | |

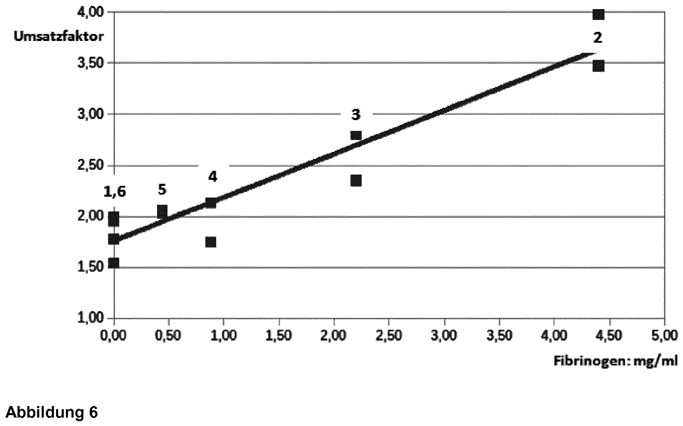

Nach einer kurzen Mischung durch Schütteln wurde die Fluoreszenzzunahme durch die Substratspaltung über jeweils 5 Minuten registriert. Die $\Delta$l/min-Werte wurden jeweils paarweise durcheinander dividiert, d.h. 1/2 , 3/4 , bis 11/12 . Dabei fiel auf, dass die Enzymaktivität innerhalb einer Stunde von Well 1 bis 11 von 100% auf 15% abnahm. Zwischen den miteinander verrechneten Wertepaaren lagen aber nur 5 Minuten Zeitdifferenz und es ergab sich eine sinnvolle Abhängigkeit der Umsatzfaktoren von der Fibrinogenkonzentration. Die Methode ist also in der Lage, auch große Fehler in den Enzymaktivitäten auszugleichen, sofern die Differenzen zwischen den verrechneten Kompartimenten klein sind. Die Ergebnisse sind in Abbildung 6 gezeigt.

Die Instabilität des verdünnten Enzyms beruhte auf Adsorption an der Glasoberfläche der Injektorspritze. Zusatz von 0,1% PEG6000 und 0,25M NaCl stabilisierten das Enzym.

Bei Fluoreszenzmessungen ist die Abhängigkeit der Fluoreszenzintensität von der Anwesenheit von löschenden Substanzen (Quenchern) häufig von Nachteil. Da in der erfindungsgemäßen Ausführung der Fluorophor in beiden Kompartimente identisch beeinflusst wird, wird ein potentieller Lösch-Effekt durch die Verrechnung kompensiert.

**Abspaltung** von Phenylendiamin / Photometrischer und elektrochemischer Nachweis

Thrombin spaltet auch Z-Gly-Pro-Arg-p-Phenylendiamin. Das Produkt konnte durch oxidative Kupplung mit $\alpha$-Naphthol in Gegenwart von Hexacyanoferrat III nachgewiesen werden. Es ergab sich ein violetter Farbstoff mit $\lambda_{max}$ = 565nm. Die Michaelis-Menten-Konstante bei pH 8 (Puffer wie für die Fluoreszenzmessung + 2% Triton X100) betrug 6µM.

Alternativ kann das Phenylendiamin auch elektrochemisch oxidiert und der Anstieg des Stroms über die Zeit als Maß für die Enzymaktivität verwendet werden. Mit hoher und niedrigerer Substratkonzentration lässt sich Fibrinogen ähnlich messen wie mit der Fluoreszenz. Elektrochemische Messungen bilden den Stofftransport zur Elektrodenfläche ab. Dieser ist proportional zur Konzentration und zum Diffusionskoeffizienten. Gerade in biologischem Probematerial ist dieser häufig sehr variabel, z.B. durch Makromoleküle oder gar Blutzellen/Hämatokrit. Deswegen gibt es z.B. in der Anwendung für Blutglucosetests eine Fülle von Zusatzmessungen, z.B. komplexe Impedanz, die diese Variationen erfassen und korrigieren. Bei der erfindungsgemäßen Methode sind die Viskosität und der Hämatokrit in beiden Kompartimenten gleich und werden durch die Verrechnung kompensiert.

Beschreibung der Abbildungen

Abbildung 1 zeigt den Verlauf der Funktion U([I]) für $[S_2]$ = $K_M$ und $[S_1]$ = 10*$K_M$.

Abbildung 2 stellt die Geschwindigkeit der Umsetzung von BAPNA (y-Achse) in Abhängigkeit von der Fibrinogenkonzentration (x-Achse) dar. Gezeigt sind vier verschiedene Messreihen, wobei sowohl die Konzentration des signalerzeugenden Substrats als auch die Konzentration des Enzyms variiert wurden.

Abbildung 3 zeigt die Abhängigkeit des aus den in Abbildung 2 gezeigten Daten berechneten Umsatzfaktors von der Fibrinogenkonzentration. Gezeigt sind die Umsatzfaktoren bei zwei verschiedenen Enzymkonzentrationen sowie die daraus ermittelte Ausgleichsgerade.

Abbildung 4 zeigt die normierte Umsatzgeschwindigkeit V/$V_{max}$ in Abhängigkeit von der normierten Substratkonzentration [S]/$K_M$ (logarithmische Skala). Gezeigt ist die Abhängigkeit für verschiedene Analytkonzentrationen.

Abbildung 5 die Abhängigkeit des Umsatzfaktors (y-Achse) von der normierten Analytkonzentration [I]/$K_I$. Gezeigt ist die Abhängigkeit für verschiedene Substratkonzentrationen.

Abbildung 6 zeigt den Umsatzfaktor (y-Achse) in Abhängigkeit von der Fibrinogenkonzentration (x-Achse). Die Zahlen 1 bis 6 verweisen auf die im Pipettierschema gezeigten Messreihen, wobei "1" in Abbildung 6 der Verrechnung der Messreihen 1 und 2, "2" der Verrechnung der Messreihen 3 und 4, "3" der Verrechnung der Messreihen 5 und 6, "4" der Verrechnung der Messreihen 7 und 8, "5" der Verrechnung der Messreihen 9 und 10, und "6" der Verrechnung der Messreihen 11 und 12 entspricht.

**Patentansprüche**

1. Verfahren zur Bestimmung des Gehalts eines Analyten in einer Probe umfassend die folgenden Schritte:

   a. Bereitstellen mindestens einer Analyselösung,

      i. wobei die mindestens eine Analyselösung jeweils ein Enzym, ein signalerzeugendes Substrat und einen bekannten Anteil der zu analysierenden Probe umfasst, und
      ii. wobei das Enzym sowohl den Analyten als auch das signalerzeugende Substrat umsetzen kann, so dass Analyt und signalerzeugendes Substrat um die Umsetzung durch das Enzym konkurrieren,

   b. Detektion zweier durch enzymkatalysierte Umsetzung in der mindestens einen Analyselösung erzeugter Signale S1 und S2, wobei die Signale

      i. durch enzymkatalysierte Umsetzung einer höheren Konzentration des signalerzeugenden Substrats (Signal S1) und durch enzymkatalysierte Umsetzung einer niedrigeren Konzentration des signalerzeugenden Substrats (Signal S2) in zwei separaten Analyselösungen, oder
      ii. durch enzymkatalysierte Umsetzung des signalerzeugenden Substrats (Signal S1) und durch enzymkatalysierte Umsetzung des Analyten (Signal S2), oder
      iii. durch enzymkatalysierte Umsetzung eines ersten signalerzeugenden Substrats (Signal S1) und durch enzymkatalysierte Umsetzung eines zweiten signalerzeugenden Substrats (Signal S2) erzeugt werden,

   c. Berechnung eines Umsatzfaktors aus den Signalen, wobei der Umsatzfaktor als Quotient der Anfangsgeschwindigkeiten $v_0(S2)/v_0(S1)$ oder als dessen Kehrwert $v_0(S1)/v_0(S2)$ berechnet wird, und
   d. Bestimmung des Gehalts des Analyten in der Probe mit Hilfe des Umsatzfaktors.

2. Verfahren nach Anspruch 1, wobei in Schritt a eine erste Analyselösung A1 und eine zweite Analyselösung A2 bereitgestellt werden.

3. Verfahren nach Anspruch 2, wobei die Konzentration des signalerzeugenden Substrats in der ersten Analyselösung A1 mindestens doppelt so hoch ist wie die Konzentration des signalerzeugenden Substrats in der zweiten Analyselösung A2.

4. Verfahren nach Anspruch 2 oder 3, wobei die Konzentration des signalerzeugenden Substrats in der ersten Analyselösung A1 mindestens 80% der zur Sättigung des Enzyms benötigten Konzentration beträgt.

5. Verfahren nach mindestens einem der vorhergehenden Ansprüche, wobei das Enzym Thrombin und der Analyt Fibrinogen ist.

6. Verfahren nach einem der vorstehenden Ansprüche, wobei die Analyselösung in Küvetten und/oder Mikrotiterplatten bereitgestellt wird.

7. Verwendung eines Teststreifens zur Durchführung des Verfahrens nach mindestens einem der vorhergehenden Ansprüche, wobei der Teststreifen mindestens eine das Enzym enthaltende Enzymschicht und mindestens eine das signalerzeugende Substrat enthaltende Substratschicht umfasst.

8. Verwendung eines Testsystems zur Durchführung des Verfahrens nach mindestens einem der Ansprüche 1 bis 6, wobei das Testsystem einen Teststreifen, der mindestens eine das Enzym enthaltende Enzymschicht und mindestens eine das signalerzeugende Substrat enthaltende Substratschicht umfasst, sowie einen Detektor zur Detektion der Signale S1 und S2 umfasst.

9. Verwendung eines Kits zur Durchführung des Verfahrens nach einem der Ansprüche 1 bis 6, wobei das Kit mindestens eine Enzymzubereitung und mindestens eine Zubereitung mindestens eines signalerzeugenden Substrats umfasst.

10. Verwendung nach Anspruch 9, weiterhin umfassend Anweisungen zur Durchführung des Verfahrens nach einem der Ansprüche 1 bis 6.

11. Verwendung nach einem der Ansprüche 9 bis 10, wobei das Kit weiterhin mindestens eine Küvette und/oder mindestens eine Mikrotiterplatte umfasst.

12. Verwendung nach einem der Ansprüche 9 bis 11, wobei die Enzymzubereitung und die Zubereitung des mindestens einen signalerzeugenden Substrats in getrennten Behältnissen bereitgestellt sind.

**Claims**

1. A method for the determination of the content of an analyte in a sample comprising the following steps:

   a. providing at least one analysis solution,

      i. wherein the at least one analysis solution comprises each of an enzyme, a signal-generating substrate and a known portion of the sample to be analyzed, and
      ii. wherein the enzyme is able to convert both, the analyte and also the signal-generating substrate, so that analyte and signal-generating substrate compete for the conversion by the enzyme,

   b. detection of two signals S1 and S2 generated by enzyme-catalyzed conversion in the at least one analysis solution, wherein the signals are generated

      i. by enzyme-catalyzed conversion of a higher concentration of the signal-generating substrate (signal S1) and by enzyme-catalyzed conversion of a lower concentration of the signal-generating substrate (signal S2) in two separate analysis solutions, or
      ii. by enzyme-catalyzed conversion of the signal-generating substrate (signal S1) and by enzyme-catalyzed conversion of the analyte (signal S2), or
      iii. by enzyme-catalyzed conversion of a first signal-generating substrate (signal S1) and by enzyme-catalyzed conversion of a second signal-generating substrate (signal S2),

   c. calculation of a conversion factor from the signals, wherein the conversion factor is calculated as quotient of the initial rates $v_0(S2)/v_0(S1)$ or as its inverse value $v_0(S1)/v_0(S2)$, and
   d. determination of the content of the analyte in the sample with the help of the conversion factor.

2. The method according to claim 1, wherein in step a, a first analysis solution A1 and a second analysis solution A2 are provided.

3. The method according to claim 2, wherein the concentration of the signal-generating substrate in the first analysis solution A1 is at least twice as high as the concentration of the signal-generating substrate in the second analysis solution A2.

4. The method according to claim 2 or 3, wherein the concentration of the signal-generating substrate in the first analysis solution A1 is at least 80% of the concentration being required for the saturation of the enzyme.

5. The method according to at least one of the preceding claims, wherein the enzyme is thrombin and the analyte is fibrinogen.

6. The method according to one of the preceding claims, wherein the analysis solution is provided in cuvettes and/or microtiter plates.

7. A use of a testing strip for conducting the method according to at least one of the preceding claims, wherein the testing strip comprises at least one enzyme layer containing the enzyme and at least one substrate layer containing the signal-generating substrate.

8. A use of a test system for conducting the method according to at least one of claims 1 to 6, wherein the test system comprises a testing strip which comprises at least one enzyme layer containing the enzyme and at least one substrate layer containing the signal-generating substrate, as well as a detector for detecting the signals S1 and S2.

9. A use of a kit for conducting the method according to one of claims 1 to 6, wherein the kit comprises at least one enzyme preparation and at least one preparation of at least one signal-generating substrate.

10. The use according to claim 9, further comprising instructions for conducting the method according to one of claims 1 to 6.

11. The use according to one of claims 9 to 10, wherein the kit further comprises at least one cuvette and/or at least one microtiter plate.

12. The use according to one of claims 9 to 11, wherein the enzyme preparation and the preparation of the at least one signal-generating substrate are provided in separated receptacles.


**Revendications**

1. Procédé de détermination de la quantité d'un analyte contenue dans un échantillon, le procédé comprenant les étapes suivantes :

   a. fournir au moins une solution d'analyse,

   i. dans lequel l'au moins une solution d'analyse comprend une enzyme, un substrat générateur de signal et une proportion connue de l'échantillon à analyser, et
   ii. dans lequel l'enzyme peut transformer aussi bien l'analyte que le substrat générateur de signal de sorte que l'analyte et le substrat générateur de signal entrent en compétition pour la transformation par l'enzyme,

   b. détecter deux signaux S1 et S2 générés par transformation par catalyse enzymatique dans l'au moins une solution d'analyse, les signaux étant générés

   i. par transformation par catalyse enzymatique d'une concentration plus élevée du substrat générateur de signal (signal S1) et par transformation par catalyse enzymatique d'une concentration plus faible du substrat générateur de signal (signal S2) dans deux solutions d'analyse séparées, ou
   ii. par transformation par catalyse enzymatique du substrat générateur de signal (signal S1) et par transformation par catalyse enzymatique de l'analyte (signal S2), ou
   iii. par transformation par catalyse enzymatique d'un premier substrat générateur de signal (signal S1) et par transformation par catalyse enzymatique d'un deuxième substrat générateur de signal (signal S2),

   c. calculer un facteur de transformation à partir des signaux, le facteur de transformation étant calculé comme le quotient des vitesses initiales $v_0(S2)/v_0(S1)$ ou comme sa valeur réciproque $v_0(S1)/v_0(S2)$, et
   d. déterminer la quantité d'analyte contenue dans l'échantillon à l'aide du facteur de transformation.

2. Procédé selon la revendication 1, dans lequel une première solution d'analyse A1 et une deuxième solution d'analyse A2 sont fournies à l'étape a.

3. Procédé selon la revendication 2, dans lequel la concentration du substrat générateur de signal dans la première solution d'analyse A1 étant au moins deux fois plus élevée que la concentration du substrat générateur de signal dans la deuxième solution d'analyse A2.

4. Procédé selon la revendication 2 ou 3, dans lequel la concentration du substrat générateur de signal dans la première solution d'analyse A1 est égale à au moins 80 % de la concentration nécessaire pour saturer l'enzyme.

5. Procédé selon l'une au moins des revendications précédentes, dans lequel l'enzyme est la thrombine et l'analyte étant le fibrinogène.

6. Procédé selon l'une des revendications précédentes, dans lequel la solution d'analyse étant fournie dans des cuvettes et/ou des plaques de micro-titrage.

7. Utilisation d'une bandelette de test pour la mise en œuvre du procédé selon l'une au moins des revendications précédentes, dans laquelle la bandelette de test comprend au moins une couche d'enzyme contenant l'enzyme et au moins une couche de substrat contenant le substrat générateur de signal.

8. Utilisation d'un système de test pour la mise en œuvre du procédé selon l'une au moins des revendications 1 à 6, dans laquelle le système de test comprend une bandelette de test qui comprend au moins une couche d'enzyme contenant l'enzyme et au moins une couche de substrat contenant le substrat générateur de signal, et un détecteur destiné à détecter les signaux S1 et S2.

9. Utilisation d'un kit destiné à la mise en œuvre du procédé selon l'une des revendications 1 à 6, dans laquelle le kit comprend au moins une préparation enzymatique et au moins une préparation d'au moins un substrat générateur de signal.

10. Utilisation selon la revendication 9, comprenant en outre des instructions pour mettre en œuvre le procédé selon l'une des revendications 1 à 6.

11. Utilisation selon l'une des revendications 9 à 10, dans laquelle le kit comprend en outre au moins une cuvette et/ou au moins une plaque de micro-titrage.

12. Utilisation selon l'une des revendications 9 à 11, dans laquelle la préparation enzymatique et la préparation de l'au moins un substrat générateur de signal sont fournies dans des récipients séparés.

**Abbildung 1**

**Abbildung 2**

**Umsatzfaktor**

Abbildung 3

Abbildung 4

Abbildung 5

EP 3 538 664 B1

**Abbildung 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013053953 A1

- EP 0357400 A2

**In der Beschreibung aufgeführte Nicht-Patentliteratur**

- **ROSSAINT et al.** *Critical Care,* 2016, vol. 20, 100
- **FREEMAN ; BACHAS.** Fiber-optic biosensor with fluorescence detection based on immobilized alkaline phosphatase. *Biosensors & Bioelectronics,* 1992, vol. 7, 49-55

- **KENSLER et al.** A facile enzymatic technique for the estimation of nanomolar concentrations of N-phosphonoacetyl-L-aspartic acid in plasma. *Journal of Biochemical and Biophysical Methods,* 1980, vol. 2, 29-35
- **Z.B. MATHUR.** *Biochemistry,* 1993, vol. 32, 7568
- **J. HÖNES.** *Habilitationsschrift Universität Stuttgart,* 1985, 59-61